# EUROPÄISCHE PATENTSCHRIFT

(11) **EP 3 401 299 B1**
(45) Veröffentlichungstag und Bekanntmachung des Hinweises auf die Patenterteilung: **03.11.2021**
(21) Anmeldenummer: 17400024.0
(22) Anmeldetag: 12.05.2017
(51) Int. Cl.: C07C 29/152, C07C 31/04, B01J 8/04, B01J 8/06

(54) **REAKTOR ZUM DURCHFÜHREN EXOTHERMER GLEICHGEWICHTSREAKTIONEN**
REACTOR FOR CARRYING OUT EXOTHERMIC EQUILIBRIUM REACTIONS
RÉACTEUR DESTINÉ À EFFECTUER DES RÉACTIONS D'ÉQUILIBRE EXOTHERMIQUES

(43) Veröffentlichungstag der Anmeldung: 14.11.2018
(73) Patentinhaber: L'AIR LIQUIDE, SOCIÉTÉ ANONYME POUR L'ÉTUDE ET L'EXPLOITATION DES PROCÉDÉS GEORGES CLAUDE, 75007 Paris (FR)
(72) Erfinder: Ochs, Andreas, 61350 Bad Homburg (DE); Castillo-Welter, Frank, 61381 Friedrichsdorf (DE); Haag, Stéphane, 60598 Frankfurt am Main (FR); Frind, Robert, 64331 Weiterstadt (DE); Schuhmann, Timm, 63067 Offenbach (DE); Wagner, Marc, 94100 Saint Maur des Fosses (FR); Valentin, Solène, 78230 LE PECQ (FR)
(74) Vertreter: Dropsch, Holger

(56) Entgegenhaltungen:
- EP-A2- 0 483 919
- EP-A2- 0 494 350
- WO-A1-2005/115607
- WO-A1-2015/030578
- WO-A1-2015/193440
- WO-A2-2007/096699
- CN-A- 106 380 376
- CN-A- 106 518 609
- US-A1- 2010 280 136
- US-A1- 2016 159 714

## Beschreibung

### Gebiet der Erfindung

Die Erfindung betrifft einen Reaktor zum Durchführen exothermer Gleichgewichtsreaktionen, bei denen ein gasförmiges Einsatzgemisch an einem festen Katalysator mindestens teilweise zu einem Produktgemisch umgesetzt wird. Ein Anwendungsbeispiel ist die Durchführung der Methanolsynthese durch heterogen-katalysierte Umsetzung von Wasserstoff und Kohlenoxide umfassendem Synthesegas an festen Katalysatoren.

### Stand der Technik

Reaktoren zur Durchführung exothermer Gleichgewichtsreaktionen sind der Fachwelt seit langem bekannt. Eine industriell besonders wichtige Reaktion dieses Typs ist die Methanolsynthese durch heterogen-katalytische Umsetzung von Synthesegas, also Gemischen von Wasserstoff und Kohlenoxiden. In Ullmann's Encyclopedia of Industrial Chemistry, Sixth Edition, 1998 Electronic Release, Kapitel "Methanol", Unterkapitel 5.2 "Synthesis" werden verschiedene Grundverfahren zur Herstellung von Methanol durch katalytische Umsetzung von Wasserstoff und Kohlenstoffoxide enthaltendem Synthesegas beschrieben, bei denen solche Reaktoren eingesetzt werden.

Ein moderneres, zweistufiges Verfahren zur Herstellung von Methanol ist beispielsweise aus der europäischen Patentschrift EP 0 790 226 B1 bekannt. Das Methanol wird in einem Kreisprozess hergestellt, bei dem eine Mischung von frischem und teilweise reagiertem Synthesegas zunächst einem wassergekühlten Reaktor und dann einem gasgekühlten Reaktor zugeführt wird, in welchen das Synthesegas jeweils an einem kupferbasierten Katalysator zu Methanol umgesetzt wird. Das in dem Prozess hergestellte Methanol wird von dem zurückzuführenden Synthesegas abgeschieden, welches dann als Kühlmittel im Gegenstrom durch den gasgekühlten Reaktor hindurchgeführt und auf eine Temperatur von 220 bis 280°C vorgewärmt wird, bevor es in den ersten Synthesereaktor eingebracht wird. Ein Teil des zurückzuführenden Synthesegases wird als Spülstrom (sog. Purge) aus dem Verfahren entfernt, um zu verhindern, dass sich Inertkomponenten innerhalb des Synthesekreislaufes anreichern. Diese Maßnahme wird auch in der deutschen Offenlegungsschrift DE 2934332 A1 und der europäischen Patentanmeldung EP 1016643 A1 gelehrt.

In der wassergekühlten Reaktorstufe wird üblicherweise der Hauptumsatz des Synthesegases (CO, CO2, H2) erzielt und der größte Teil der Reaktionswärme abgeführt, während in der gasgekühlten Stufe bei milderen Bedingungen ein dennoch erheblicher Teil des Synthesegases umgesetzt wird.

In manchen Anlagenkonfigurationen wird zusätzlich eine Zwischenkondensationsstufe zwischen den beiden Reaktionsstufen vorgesehen, um den Anteil der gebildeten Reaktionsprodukte (vorwiegend Methanol und Wasser) im Einsatzgas zur zweiten Reaktionsstufe zu verringern und damit den erzielbaren Umsatz der Edukte weiter zu erhöhen. Eine solche Anlagenkonfigurationen wird beispielsweise in der deutschen Patentschrift DE 10 2008 049 622 B4 gelehrt.

Bei dem wassergekühlten Reaktor (WCR) handelt es sich üblicherweise um einen Röhrenreaktor mit entsprechenden Rohrplatten, bei dem der Katalysator in die Rohre gefüllt wird, während die Kühlung mittels siedendem Wasser bzw. Dampferzeugung auf der Mantelseite um die Rohre erfolgt. Im gasgekühlten Reaktor (GCR) erfolgt die Kühlung mit dem Einsatzgas, welches durch die Rohre geführt wird und sich auf dem Weg zur ersten Reaktionsstufe (WCR) erwärmt, während der Katalysator um die Rohre gefüllt wird und die Reaktion auf der Mantelseite des GCR stattfindet. Die Reaktionsstufen sind hinsichtlich ihrer Nennweite mit großen bzw. sehr großen Rohrleitungen verbunden; je nach Anlagenkapazität sind Rohrdurchmesser von bis zu 1 m möglich. Dies ist vor allem auf die großen Gasmengen zurückzuführen, die zu der zweiten Stufe zurückgeführt (Recycle-Gas) und dem Frischgas, also frischem Syn-thesegas aus der Gaserzeugung, zugemischt werden. Das sich ergebende Gas-gemisch aus Rückführungsgas und Frischgas wird nach erfolgter Vorwärmung im GCR der ersten Reaktionsstufe (WCR) zugeführt. Die Rückführungsgasmenge (Recycle-Gas) ist üblicherweise deutlich größer als die Frischgasmenge und ist vom erzielten Umsatz in der Reaktorsektion abhängig. Das Rückführverhältnis RR (RR=R/F) aus Rückführungsgasmenge (R) zu Frischgasmenge (F) liegt oft über 2 und in einigen Fällen sogar über 3,5. Je niedriger der Umsatz von Synthesegas durch die Reaktorsektion per Durchgang ist, um so höher ist das erforderliche Rückführverhältnis RR, um eine hinreichende Ausbeute zu erzielen.

Damit erhöht sich die umlaufende Gasmenge entsprechend, was die Belastung der Reaktoren erhöht und größere Rohrnennweiten der verbindenden Rohrleitungen bedarf und auch zu einem höherem Bedarf an Kompressionsenergie (höhere Durchflussmenge und Druckverlust) führt.

### Weiterer einschlägiger Stand der Technik

Das Patentdokument CN 106 518 609 A offenbart eine Methanolsynthesevorrichtung und ein Methanolsyntheseverfahren und zielt darauf ab, die Probleme des hohen Energieverbrauchs und der geringen Effizienz im Stand der Technik zu lösen. Die Methanolsynthesevorrichtung umfasst N Reaktionsstrukturen, die sequentiell von vorne nach hinten angeordnet sind. Jede Reaktionsstruktur umfasst einen Methanolsynthesereaktor und einen Gas-Flüssigkeits-Abscheider, der Methanolsynthesereaktor umfasst einen Reaktanteneinlass und einen Materialauslass, und der Gas-Flüssigkeits-Abscheider umfasst einen Materialeinlass, einen Gasphasenauslass und einen Flüssigphasenauslass. In jeder Reaktionsstruktur ist der Materialauslass des Methanolsynthesereaktors mit dem Materialeinlass des Gas-Flüssigkeits-Abscheiders verbunden. Bei jeweils zwei benachbarten Reaktionsstrukturen ist der Gasphasenauslass des Gas-Flüssig-Abscheiders der früheren Reaktionsstruktur mit dem Reaktanteneinlass des Methanolsynthesereaktors der späteren Reaktionsstruktur verbunden. N ist eine positive ganze Zahl größer oder gleich 2. Die Vorrichtung hat einen niedrigen Energieverbrauch und einen hohen Wirkungsgrad. Die Methanolsynthesemethode kann für die Synthese von Methanol mit Hilfe der Vorrichtung verwendet werden. Der Energieverbrauch kann reduziert und die Methanolsyntheserate kann durch die Verwendung der Methode erhöht werden.

Das Patentdokument CN 106 380 376 A offenbart eine Erfindung auf dem technischen Gebiet der Methanolsynthese, insbesondere ein Synthesesystem und eine Aufbereitungsanlage. Das Synthesesystem umfasst eine erste Synthesebaugruppe und eine zweite Synthesebaugruppe, wobei die erste Synthesebaugruppe und die zweite Synthesebaugruppe miteinander kommunizieren; die erste Synthesebaugruppe dient zur Kommunikation mit einer externen Synthesegasspeichereinrichtung. Gemäß dem erfindungsgemäßen Synthesesystem sind die erste Synthesebaugruppe und die zweite Synthesebaugruppe in einem seriellen Verbindungsmodus verbunden; nicht umgesetztes Synthesegas in der ersten Synthesebaugruppe wird kontinuierlich in die zweite Synthesebaugruppe eingeleitet, um eine Reaktion für die Synthese durchzuführen; das nicht umgesetzte muss nach der Druckbeaufschlagung durch einen Zirkulationskompressor nicht wieder zur ersten Synthesebaugruppe befördert werden. Daher hat das erfindungsgemäße Synthesesystem die Vorteile, dass ein Zirkulationskompressor mit einem komplizierten inneren Aufbau und großem Stromverbrauch entfällt, so dass die Betriebskosten des Synthesesystems niedriger sind; der Betrieb und die Wartung sind einfach.

Das Patentdokument EP 0 483 919 A2 offenbart ein Verfahren zur Herstellung von Methanol durch Umsetzung eines Wasserstoff und Kohlenmonoxid enthaltenden Gasgemisches in Gegenwart einer Katalysatorzusammensetzung in einem Wirbelbett unter Kühlung, dadurch gekennzeichnet, dass die Reaktion in einer Vielzahl von Katalysator-Wirbelbettreaktoren in Serie unter zwischenzeitlicher Entfernung von Methanol aus dem Reaktionsgemisch durchgeführt wird.

Das Patentdokument US 2010/280136 A1 offenbart ein Verfahren zum Ausführen von mindestens zwei Einheitsoperationen in Reihe, wobei das Verfahren den folgenden Schritt umfasst: (a) Leiten eines Zufuhrstroms in eine integrierte Anordnung, die eine erste Mikrokanal-Einheitsoperation auf mindestens eine Chemikalie des Zufuhrstroms umfasst, um einen verteilten Ausgangsstrom zu erzeugen, der die erste Mikrokanal-Einheitsoperation in einem ersten Satz diskreter Mikrokanäle verlässt, die den Fluss durch die diskreten Mikrokanäle isolieren; und (b) Leiten des verteilten Ausgabestroms der ersten Mikrokanal-Einheitsoperation in eine zweite Mikrokanal-Einheitsoperation als verteilter Eingabestrom, um die Isolierung der Strömung zwischen dem ersten Satz diskreter Mikrokanäle fortzusetzen, und Durchführen mindestens einer Operation an mindestens einer Chemikalie des Eingabestroms, um einen Produktstrom zu erzeugen, der die zweite Mikrokanal-Einheitsoperation verlässt, wobei die erste Mikrokanal-Einheitsoperation und die zweite Einheitsoperation sich ein Gehäuse teilen.

Das Patentdokument WO 2015/030578 A1 offenbart ein Verfahren und einen Reaktor zur Herstellung eines flüssigen Methanol- und Wasserkondensats aus einer gasförmigen CO2- und H2-Beschickung oder aus einer gasförmigen CO2-, CO- und H2-Beschickung, oder ein Verfahren zur Herstellung eines flüssigen Methanolkondensats aus einer gasförmigen CO- und H2-Beschickung. Der Reaktor hat eine separate Reaktionszone und eine Kondensationszone. Die Erfinder haben gezeigt, dass durch Abtrennung eines flüssigen Kondensats in einer Kondensationszone, die innerhalb des Reaktors angeordnet ist, von den gasförmigen Reaktionsprodukten und durch Ermöglichung eines konvektiven Stoffaustauschs zwischen der Reaktionszone und der Kondensationszone unter erzwungenen und natürlichen Konvektionsbedingungen der gasförmige Einsatz vollständig in ein flüssiges Produkt umgewandelt werden kann, das Methanol mit einer hohen Selektivität der Reaktanten gegenüber Methanol umfasst.

Das Patentdokument EP 0 494 350 A2 offenbart ein Verfahren und eine Anlage zur Synthese von Methanol aus Wasserstoff, Kohlenmonoxid und Kohlendioxid unter Druck mit mindestens einer Stufe, die einen Reaktor enthält, soll die Ausbeute erhöhen und die Möglichkeit der Steuerung verbessern. Dies wird erreicht, indem mit jedem Reaktor mindestens zwei Synthesestufen eingesetzt werden und die letzte Synthesestufe mit einem Kreisgasverdichter ausgerüstet wird, wobei die vorgeschalteten Synthesestufen mittels dieses Kreisgasverdichters zu Teilkreisläufen unter Druck gesetzt werden.

Das Patentdokument US 2016/159714 A1 offenbart ein Design für ein mobiles System, das Fackelgas oder Erdgas unter Verwendung von Luft ohne Dampf reformiert, um direkt Dimethylether (DME), einen Dieselersatzstoff, zu erzeugen. Das System reformiert zunächst das Luft-Methan-Gemisch bei atmosphärischem Druck und komprimiert dann das resultierende CO-Wasserstoff-Stickstoff-Gasgemisch auf bis zu 600 psi und leitet es durch einen kombinierten Reaktor, der das Gasgemisch direkt zu Dimethylether umsetzt. Der Stickstoff wird vom System wieder an die Atmosphäre abgegeben. DME ist ein hervorragender Dieselkraftstoff und kann verwendet werden, um den wesentlich teureren und schmutzigeren Dieselkraftstoff auf Erdölbasis zu ersetzen und gleichzeitig ein kritisches Problem mit dem Abfackeln zu lösen.

Das Patentdokument WO 2015/193440 A1 offenbart ein Verfahren, das die folgenden Schritte umfasst: Zuführen eines Synthesegasstroms mit Modul M zu einem Methanolkreislauf, Durchleiten des Synthesegases in dem Methanolkreislauf durch einen ersten Methanolreaktor, Gewinnen eines ersten Abflusses aus dem ersten Methanolreaktor, Kühlen des ersten Abflusses und Kondensieren mindestens eines Teils des erzeugten Methanols, Trennen des ersten gekühlten Abflusses in mindestens einen ersten Rohmethanolstrom und einen ersten nicht umgesetzten Strom, Erhitzen des ersten nicht umgesetzten Stroms, Leiten des ersten erhitzten nicht umgesetzten Stroms durch einen zweiten Methanolreaktor, Gewinnen eines zweiten Abflusses aus dem zweiten Methanolreaktor, Trennen des zweiten Abflusses in mindestens einen zweiten RohMethanolstrom und einen zweiten nicht umgesetzten Strom, und Recyceln des zweiten nicht umgesetzten Stroms zum Synthesegasstrom.

Das Patentdokument WO 2007/096699 A2 offenbart ein Mehrfachreaktionssystem für die Herstellung von Chemikalien durch gleichgewichtsbegrenzte Reaktionen unter Verwendung von Plattenwärmetauschern oder Wärmetauschern mit erweiterter Oberfläche. Die Wärmetauscher kühlen die Reaktionsprodukte ab, um das in den Reaktionsprodukten enthaltene Methanol für die Abtrennung zu kondensieren, und erwärmen auch die ankommenden Einsatzreaktanten vor dem Eintritt der Reaktanten in einen Reaktor, der für die Herstellung von Methanol verwendet wird. Die verschiedenen Reaktoren, Wärmetauscher und Separatoren können als getrennte Zonen innerhalb der geschlossenen Gefäße gebildet werden, wodurch die Notwendigkeit für separat konstruierte Reaktoren, Wärmetauscher und Separatoren entfällt. Für eine effiziente Kühlung und Methanolabtrennung können Mehrstrom-Plattenwärmetauscher mit großer Oberfläche eingesetzt werden. Das Mehrfachreaktionssystem kann auch für die Rückgewinnung von Methanol aus einem Ab- oder Spülgasstrom verwendet werden, wobei mehrere Reaktoren, mehrere Plattenwärmetauscher oder Wärmetauscher mit erweiterter Oberfläche und mehrere Separatoren als Ersatz für oder in Verbindung mit einem herkömmlichen Methanolsynthesekreislauf eingesetzt werden.

Das Patentdokument WO 2005/115607 A1 offenbart ein Verfahren zur Durchführung von heterogen katalytischen exothermen Gasphasenreaktionen bei hoher Temperatur und hohem Druck, bei dem das Synthesegas, bestehend aus einem Gemisch von Make-up-Gas und/oder von Recycle-Gas, durch mindestens zwei Synthesestufen geleitet wird, die in Reihe zu einem Synthesesystem geschaltet sind. Die Produktgase aus den Synthesestufen, mit Ausnahme der letzten Stufe, werden in mindestens zwei Teilströme aufgeteilt. Ein Teilstrom wird gekühlt, bis das Produkt auskondensiert ist, und das produkthaltige Kondensat wird von den gasförmigen Bestandteilen getrennt. Anschließend werden die gasförmigen Bestandteile mit dem warmen Teil des Produktgases zusammengeführt, um die Eintrittstemperatur der nächsten Synthesestufe zu erreichen. Zusätzlich kann der Teilstrom, aus dem das Produkt auskondensiert und abgezogen wurde, aufgeheizt werden, bevor er wieder mit dem warmen Teil des Produktgases vermischt wird. Nach dem Aufheizen ist die Temperatur geringer als die des warmen Produktgasanteils, und die Wärme, die zum Aufheizen des Teilstroms, aus dem das Produkt auskondensiert wurde, verwendet wurde, wird diesem Teilstrom beim Abkühlen zumindest teilweise entzogen.

### Beschreibung der Erfindung

Die Aufgabe der vorliegenden Erfindung besteht daher darin, einen Reaktor anzugeben, der die beschriebenen Nachteile der aus dem Stand der Technik bekannten Reaktoren nicht aufweist und der insbesondere einen hohen Umsatz bezüglich der Zielprodukte der exothermen Reaktion pro Reaktordurchgang sowie die Möglichkeit bietet, die Reaktionsbedingungen entlang der Längskoordinate des Reaktors nachzuführen und somit zu optimieren, was beispielsweise im Falle der Methanolsynthese zu einer Reduzierung des Rückführverhältnisses auf kleinere Werte führt, wie sie bei Verwendung der aus dem Stand der Technik bekannten Reaktoren bekannt sind.

Diese Aufgabe wird durch einen Reaktor mit den Merkmalen des Anspruchs 1 gelöst. Weitere Ausgestaltungen der Erfindung ergeben sich aus den Unteransprüchen.

### Erfindungsgemäßer Reaktor:

Reaktor zum Durchführen exothermer Gleichgewichtsreaktionen, bei denen ein gasförmiges Einsatzgemisch an einem festen Katalysator mindestens teilweise zu einem Produktgemisch umgesetzt wird, das mindestens ein bei dem Reaktordruck und bei Temperaturen unterhalb der Reaktortemperatur kondensierbares, flüssiges Reaktionsprodukt enthält, umfassend mindestens zwei hintereinander geschaltete und in Fluidverbindung miteinander stehende Reaktionszellen, die in einem gemeinsamen Reaktormantel angeordnet sind, wobei jede Reaktionszelle die folgenden, hintereinander geschalteten und in Fluidverbindung miteinander stehenden Baugruppen umfasst:
(a) eine Vorwärmzone, geeignet zum Aufheizen des Einsatzgemischs oder des gasförmigen Produktstroms aus der vorgelagerten Reaktionszelle, wobei die Vorwärmzone in der ersten Reaktionszelle in Strömungsrichtung des gasförmigen Einsatzgemischs optional entfallen kann,
(b) mindestens eine Reaktionszone, enthaltend einen für die durchzuführende exotherme Gleichgewichtsreaktion aktiven Katalysator als Katalysatorschüttung in einem Katalysatorbett und eine mit dem Katalysatorbett in einer Wärmeaustauschbeziehung stehende Kühlvorrichtung,
(c) mindestens eine Kühlzone, enthaltend eine Kühlvorrichtung, geeignet zur Abkühlung des aus der Reaktionszone austretenden, teilumgesetzten und mit kondensierbarem Reaktionsprodukt beladenen, gasförmigen Produktstroms auf eine Temperatur unterhalb des Taupunktes dieses Gases,
(d) eine Abscheidezone, enthaltend eine Phasentrennvorrichtung zum Auftrennen des aus der Kühlzone austretenden Produktstroms in einen von Kondensat befreiten, gasförmigen Produktstrom und einen flüssiges Reaktionsprodukt umfassenden Kondensatstrom,
(e) Mittel zum Ausleiten des flüssiges Reaktionsprodukt umfassenden Kondensatstroms und optional Mittel zum Zuführen des Kondensatstroms zu einer Aufarbeitungsvorrichtung für das Reaktionsprodukt,
(f) Mittel zum Ausleiten des von Kondensat befreiten, gasförmigen Produktstroms und Mittel zum Zuführen dieses gasförmigen Produktstroms zu einer nachgelagerten, stromabwärts angeordneten Reaktionszelle und/oder Mittel zum Ausleiten des gasförmigen Produktstroms aus dem Reaktor.

Unter Fluidverbindung zwischen zwei Bereichen des erfindungsgemäßen Reaktors wird dabei jegliche Art von Verbindung verstanden, die es ermöglicht, dass ein Fluid, beispielsweise der Einsatzgasstrom oder der Synthesegasproduktstrom, von dem einen zu dem anderen der beiden Bereiche strömen kann, unbeachtlich etwaiger zwischengeschalteter Bereiche oder Bauteile.

Mit Wärmeaustauschbeziehung ist die Möglichkeit des Wärmeaustauschs oder der Wärmeübertragung zwischen zwei Bereichen des erfindungsgemäßen Reaktors gemeint, wobei alle Mechanismen des Wärmeaustauschs oder der Wärmeübertragung wie Wärmeleitung, Wärmestrahlung oder konvektiver Wärmetransport zum Tragen kommen können. Unter einer indirekten Wärmeaustauschbeziehung wird dabei insbesondere die Art des Wärmeaustauschs oder der Wärmeübertragung verstanden, die durch eine Wand hindurch erfolgt (sog. Wärmedurchgang), der die Etappen des Wärmeübergangs von Fluid 1 auf die Oberfläche der Wand, der Wärmeleitung durch die Wand und des Wärmeübergangs von der Oberfläche der Wand auf Fluid 2 umfasst.

Unter Mitteln zum Einleiten, Ausleiten usw. werden alle die Vorrichtungen, Vorrichtungsbestandteile, Baugruppen und Bauteile verstanden, die es ermöglichen, dass das betreffende Fluid den betrachteten räumlichen Bereich, beispielsweise einen Behälter, verlässt. Hierunter werden insbesondere Rohrleitungen, Pumpen, Verdichter, sonstige Fördervorrichtungen sowie die entsprechenden Durchtrittsöffnungen in der Behälterwand verstanden.

Unter der katalytischen Aktivität, insbesondere im Zusammenhang mit einer unterschiedlichen katalytischen Aktivität beim Vergleich zweier unterschiedlicher Katalysatoren, wird der erzielte Umsatzgrad pro Längeneinheit des Katalysatorbetts von Edukten zu Produkten verstanden. Die Aktivität wird beeinflusst von der chemischen Zusammensetzung, Dotierung, Vergiftung, verfügbaren Oberfläche etc. des Katalysatormaterials, aber auch durch die Geometrie der Katalysatorpartikel und texturellen Parametern des Katalysatorbetts, z. B. dessen Porosität oder Packungsdichte. Aufgrund der Exothermie der betrachteten Reaktionen korreliert eine hohe katalytische Aktivität mit einer hohen Wärmefreisetzung pro Längeneinheit des Katalysatorbetts.

Die in Anspruch 1. (a) erwähnte Option, dass die Vorwärmzone in der ersten Reaktionszelle in Strömungsrichtung des gasförmigen Einsatzgemischs entfallen kann, wird insbesondere dann realisiert werden, wenn eine außerhalb des erfindungsgemäßen Reaktors angeordnete und diesem vorgeschaltete Heizvorrichtung vorhanden ist, die die Einstellung der Reaktionstemperatur vor Eintritt in die erste Reaktionszone gewährleistet.

Der Erfindung liegt die Erkenntnis zugrunde, dass durch eine optimale Temperaturführung sowie wiederholtes Abführen von Produkten aus der Reaktionszone die Produktionsraten bzw. Raum-Zeit-Ausbeuten entlang des Reaktionsweges deutlich verbessert werden können. Das Temperaturprofil entlang des Reaktionsweges wird durch den Einsatz eines mehrstufigen Reaktionssystems erheblich verbessert, wodurch ein deutlich höherer Umsatz per Durchgang erzielt wird.

Auch die Nebenproduktbildung bei der Methanolsynthese verringert sich bei Verwendung des erfindungsgemäßen Reaktors gegenüber dem Stand der Technik.

Ein verbessertes Temperaturprofil im Reaktor lässt sich grundsätzlich auch mit Hilfe eines Katalysator-Layer-Managements erzielen. Dabei würde man in dem Bereich, in dem man den höchsten Umsatz (Exothermie) und damit die höchsten Temperaturen erwartet, einen weniger aktiven Katalysator einsetzen und in Bereichen, wo weniger Umsatz erwartet wird, einen aktiveren Katalysator einsetzen. Jedoch ist ein solches Katalysator-Layer-Management relativ unflexibel, da man die verschiedenen Katalysatorlagen auf Basis einer bestimmten Katalysatoraktivität und einer entsprechenden Gaszusammensetzung auswählen und festlegen muss. Die Katalysatoraktivität verändert sich jedoch über seine Laufzeit der Syntheseanlage durch seine fortschreitende Desaktivierung.

Das Layer-Management und die zugehörige Kühlung des Reaktionsbettes müssen aufeinander abgestimmt werden. Während der Katalysatorlaufzeit und der damit verbundenen Katalysatordesaktivierung ändern sich die Bedingungen und eine Anpassung der Reaktionstemperatur und der zugehörigen Kühlung/Kühltemperatur ist wünschenswert, um die Desaktivierung zumindest teilweise zu kompensieren und einen hohen Umsatz bei geringer Nebenproduktbildung sicherzustellen. Mit den aus dem Stand der Technik bekannten Reaktoren lässt sich eine Anpassung der Kühlung nur für den gesamten Reaktor vornehmen; üblicherweise desaktivieren aber nicht alle Katalysatorschichten über die Betriebszeit in gleichem Maße. Die Einstellung spezifischer Reaktionsbedingungen stellt daher immer einen Kompromiss dar.

Mit dem erfindungsgemäßen Ansatz können die Reaktionsbedingungen in den verschiedenen Reaktionszellen dagegen individuell in Abhängigkeit der Katalysatoraktivität, der Gaszusammensetzung in jeder Stufe auch über die Laufzeit angepasst werden. Damit werden ein hoher Umsatz und eine geringe Nebenproduktbildung in den verschiedenen Reaktionszellen erreicht.

Mit der optimierten Temperaturführung werden auch die Maximaltemperaturen (sowie Temperaturspitzen, sog. Hot-Spots) im Katalysatorbett reduziert. Dies wirkt sich neben der Ausschleusung des Koppelproduktes aus dem Reaktionssystem, beispielsweise von Wasser bei der Methanolsynthese, positiv auf die Katalysatorlaufzeit aus. Es ist bekannt, dass sowohl hohe Temperaturen im Katalysatorbett als auch hohe Wasserkonzentrationen im Reaktionsgas zu einer rascheren Katalysatordesaktivierung beitragen.

Mit dem vorgeschlagenen Konzept wird eine verbesserte Raum-Zeit-Ausbeute erzielt; somit kann auch die Rückführungsgasmenge (Gaskreislauf) erheblich reduziert werden. Grundsätzlich kann der Reaktor somit verkleinert werden und auch der Druckverlust reduziert sich. Durch die Reduzierung der Rückführungsgasmenge ergibt sich auch, dass die Menge und die Konzentration an akkumulierten Inertgasen, beispielsweise nicht umgesetztes Methan aus der Synthesegas-Erzeugung, im Synthesegaskreislauf deutlich verringert werden und damit den gesamten Methanol-Synthesekreislauf mit Reaktorstufen, Kreislaufverdichter und weiteren Ausrüstungen entlastet. Als Optimum kann man den Vollumsatz pro Reaktordurchgang ansehen, bei dem auf einen Synthesegaskreislauf vollständig verzichtet werden könnte und mithin keine Akkumulierung von Inertgasen mehr auftritt. Ein derartiger Ansatz ist auch für andere Einsatzgaszusammensetzungen mit hohen Inertgasanteilen (z. B. hoher Stickstoffanteil bei Synthesegaserzeugung unter Verwendung von Luft) von besonderem Interesse, da im Synthesegaskreislauf die im Kreis zu führende Inertgasmenge ansteigt.

Durch kontrolliertes Abscheiden der flüssigen Produkte sowie Temperaturkontrolle in den einzelnen Reaktionszellen wird ein Auskondensieren im Katalysatorbett vermieden und der Katalysator geschont.

Die abgeschiedenen Kondensate mit unterschiedlichen Methanolanteilen können bei unterschiedlichen Bedingungen aufgereinigt bzw. direkt als Einsatz für nachgeschaltete Verfahren verwendet werden, was zu einer Energieeinsparung bei der Destillation führt.

Um den apparativen Aufwand und die Investitionskosten gering zu halten, werden erfindungsgemäß mehrere Reaktionsstufen bzw. Reaktionszellen und auch mehrere Zwischenkondensationen sowie Kühl- und Aufheizstufen in einem Reaktor realisiert. Verbindende Rohrleitungen werden möglichst vermieden, so dass sich Investitionskosten für Rohrleitungen und der Druckverlust reduzieren und die Beanspruchung der Rohrleitungen durch thermisch-mechanische Spannungen abnimmt. Die Prozessmedien werden möglichst innerhalb des Apparates von Prozessstufe zu Prozessstufe geführt.

### Bevorzugte Ausgestaltungen der Erfindung

In einer bevorzugten Ausgestaltung des erfindungsgemäßen Reaktors umfasst die Kühlzone in Baugruppe (c) folgendes:
(c1) eine erste Kühlzone, enthaltend eine Kühlvorrichtung, geeignet zur Abkühlung des aus der Reaktionszone austretenden, teilumgesetzten und mit kondensierbarem Reaktionsprodukt beladenen, gasförmigen Produktstroms auf eine Temperatur unterhalb der Temperatur in der Reaktionszone,
(c2) eine zweite Kühlzone, enthaltend eine Kühlvorrichtung, geeignet zur weiteren Abkühlung des aus der ersten Kühlzone austretenden, teilumgesetzten und mit kondensierbarem Reaktionsprodukt beladenen, vorgekühlten gasförmigen Produktstroms auf eine Temperatur unterhalb des Taupunktes dieses Gases.

Durch die Ausgestaltung der Baugruppe (c) mit einer ersten und einer zweiten Kühlzone bestehen mehr Freiheitsgrade hinsichtlich der Durchführung der Kühlung des aus der Reaktionszone austretenden, teilumgesetzten und mit kondensierbarem Reaktionsprodukt beladenen, gasförmigen Produktstroms. So kann in der ersten Kühlzone einer Vorkühlung erfolgen, wobei aber der Taupunkt des Gases nicht unterschritten wird. Die Taupunktunterschreitung und Auskondensation erfolgt dann in der zweiten Kühlzone. Alternativ kann bereits in der ersten Kühlzone eine starke Abkühlung mit Taupunktunterschreitung erfolgen. Die zweite Kühlzone dient dann zur weiteren Kondensation noch in der Gasphase verbliebener, aber kondensierbarer Bestandteile. Schließlich können in der ersten und in der zweiten Kühlzone unterschiedliche Kühlmedien oder aber dasselbe Kühlmedium bei einem anderen Temperaturniveau verwendet werden. Hierdurch wird eine verbesserte Wärmeintegration im Hinblick auf den erfindungsgemäßen Reaktor bzw. das mit diesem durchgeführte Verfahren erreicht.

Als Wärmeträger (Heizmedium) bzw. Kühlmedium werden bevorzugt Medien eingesetzt, die sich in der Nähe ihres Siedepunktes befinden und daher leicht verdampfen (Kühlmedium) oder kondensieren (Wärmeträger). Dies sichert eine gute Wärmeabfuhr durch den guten Wärmeübergang auf der Seite des verdampfenden bzw. kondensierenden Mediums und erlaubt eine präzise Temperaturregelung über den Druck. Um unterschiedliche Temperaturbedingungen in den verschiedenen Stufen einzustellen, wird der Druck auf der Seite des Wärmeträgers bzw. Kühlmediums individuell für jede Stufe geregelt. Mit zunehmender Katalysatorlaufzeit werden die Bedingungen mittels entsprechender Einstellung des Druckes auf der Kühlmediumseite angepasst und somit die Reaktionstemperatur nachgeführt, um den Umsatz entsprechend hoch zu halten.

Bezüglich der gewünschten Reaktionsbedingungen kann bei der Methanolsynthese Wasser bzw. Wasserdampf als Wärmeträger eingesetzt werden. Jedoch zeigt sich, dass bei Verwendung von Wasser für den gewünschten Temperaturbereich relativ große Druckunterschiede eingestellt werden müssen, um einen breiten Temperaturbereich abzudecken (z. B. 250 °C: ca. 40 bar, 264 °C: ca. 50 bar). Benutzt man dagegen ein verdampfendes Wärmeträgeröl (z. B. Dowtherm A) in einem Kreislauf zur Wasserdampferzeugung, so kann man in einem sehr engen Druckbereich arbeiten und dennoch einen großen Temperaturbereich abdecken (Bsp.: 255 °C: 0,97 bar, 305 °C: 2,60 bar, entsprechend einem Temperaturbereich von 50 °C bei einem Druckunterschied von nur 1,6 bar. Dadurch kann man mit einer einfachen Wärmeträgeröl-Dampftrommel auf dem entsprechendem Anlagenniveau (ca. 20 bis 25 m) arbeiten und allein die geodätische Zulaufhöhe nutzen, um die individuellen Druck- bzw. Temperaturbereiche einzustellen.

In den Abkühlzonen und/oder Kondensationszonen kann Kühlwasser oder auch ein verdampfender Wärmeträger eingesetzt werden, während in den Heizzonen ein kondensierender oder auch flüssiger Wärmeträger zum Einsatz kommen kann.

In einer weiteren bevorzugten Ausgestaltung des erfindungsgemäßen Reaktors ist der Mantel horizontal oder vertikal gegenüber der durch die Schwerkraft vermittelten Senkrechten angeordnet ist, wobei die Reaktionszellen in beiden Fällen senkrecht von dem gasförmigen Einsatzgemisch oder dem gasförmigen Produktstrom aus der vorgelagerten Reaktionszelle durchströmt werden. Hierdurch werden Vorteile hinsichtlich der Anordnung des Katalysators in der Reaktionszelle erreicht, da dieser senkrecht von dem gasförmigen Einsatzgemisch oder dem gasförmigen Produktstrom aus der vorgelagerten Reaktionszelle durchströmt wird und demgemäß als einfache Schüttung vorliegen kann und nur an seiner Unterseite durch ein Tragrost oder ähnliche Vorrichtungen gehalten werden muss.

In alternativer Ausgestaltung des erfindungsgemäßen Reaktors ist der Mantel horizontal oder vertikal gegenüber der durch die Schwerkraft vermittelten Senkrechten angeordnet, wobei die Reaktionszellen in beiden Fällen horizontal von dem gasförmigen Einsatzgemisch oder dem gasförmigen Produktstrom aus der vorgelagerten Reaktionszelle durchströmt werden. Hier ist zwar die Fixierung des Katalysators in der Reaktionszelle aufwendiger; jedoch ergeben sich Vorteile hinsichtlich eines Katalysatorwechsels, der dann in horizontaler Richtung durch dafür vorgesehene Revisionsöffnungen im Reaktormantel vorgenommen werden kann, beispielsweise durch Absaugen.

Bei vertikaler Anordnung des Mantels wird es ferner bevorzugt, wenn die Reaktionszellen horizontal und in radialer Richtung von dem gasförmigen Einsatzgemisch oder dem gasförmigen Produktstrom aus der vorgelagerten Reaktionszelle durchströmt werden. Außer den zuvor genannten Vorteilen hinsichtlich des Katalysatorwechsels ergeben sich weitere Vorteile dadurch, dass bei dieser Anordnung längere Wege durch die Katalysatorbetten bei gleichem oder verringertem Druckverlust ermöglicht werden.

In einem weiteren Aspekt des erfindungsgemäßen Reaktors ist die Reaktionszone (b) mit Thermoblechen ausgestattet, wobei die Thermobleche aus jeweils zwei miteinander verbundenen Blechen bestehen, wobei dieser Verbund auf seiner Innenseite einen gegenüber der Außenseite dicht abschließenden Hohlraum aufweist, der von einem fluiden Kühlmedium bzw. Wärmeträger durchströmt wird, wobei der Katalysator in der Reaktionszone stückig oder partikelförmig als Festkörper-Schüttung vorliegt, die jeweils so zwischen zwei benachbarten Thermoblechen angeordnet ist, dass sie senkrecht oder horizontal von dem gasförmigen Einsatzgemisch oder dem gasförmigen Produktstrom aus der vorgelagerten Reaktionszelle durchströmt werden kann und wobei der Katalysator und das Kühlmedium in einer indirekten Wärmeaustauschbeziehung stehen. Die Thermobleche zeichnen sich dabei durch einen geringen Platzbedarf bei gleichzeitig guten Wärmeaustauscheigenschaften aus. Vorteilhaft ist weiterhin, dass sie gleichzeitig als Halte- bzw. Unterteilungsvorrichtungen für das Katalysatorbett dienen können.

In alternativer Ausgestaltung können die Vorwärmzone, die Reaktionszone oder die Kühlzonen oder mehrere dieser Baugruppen als Lamellenwärmetauscher ausgeführt werden. Die Vorteile decken sich im Wesentlichen mit denjenigen der zuvor diskutierten Ausgestaltung unter Verwendung von Thermoblechen.

Bevorzugt wird als Kühlmedium Heißkondensat aus einem Dampferzeuger verwendet, wobei das Kühlmedium mindestens einen Teil der in der Reaktionszone (b) freigesetzten Reaktionswärme aufnimmt und dabei teilverdampft wird und wobei das dabei erhaltene Kondensat-Sattdampf-Gemisch oder der Sattdampf mindestens teilweise zu dem Dampferzeuger zurückgeführt wird und/oder als Wärmeträger zu der Vorwärmzone (a) derselben Reaktionszelle geführt wird. Dieses Medium kann die Reaktionswärme, die bei der in der Reaktionszone ablaufenden, exothermen Reaktion freigesetzt wird, besonders effektiv aufnehmen, da der Phasenübergang von Flüssigkeit zu Dampf mit einer besonders großen Enthalpieänderung verbunden ist. Weitere Vorteile sind damit verbunden, wenn der dabei gebildete Sattdampf als Wärmeträger zu der Vorwärmzone derselben Reaktionszelle geführt wird. Hier wird die Verdampfungsenthalpie durch Kondensation besonders effektiv an das zu erwärmende Medium abgegeben.

Weiter bevorzugt werden Mittel umfasst, die es gestatten, dass das aus einer oder mehreren Reaktionszellen abgeführte Kondensat-Sattdampf-Gemisch oder nur der Dampfanteil mindestens teilweise zu dem Dampferzeuger zurückgeführt wird und der aus dem Dampferzeuger abgezogene Sattdampf mindestens zum Teil als Exportdampf an externe Verbraucher abgegeben wird.

In besonderer Ausgestaltung des erfindungsgemäßen Reaktors umfasst dieser Mittel, die es gestatten, dass in der Vorwärmzone (a) das Aufheizen des Einsatzgemischs oder des gasförmigen Produktstroms aus der vorgelagerten Reaktionszelle im indirekten Wärmeaustausch gegen Heißkondensat aus einem Dampferzeuger erfolgt, wobei ein abgekühlter Heißkondensatstrom erhalten wird. Mittel der genannten Art können Rohrleitungen, Regeleinrichtungen wie Ventile sowie andere für diesen Zweck geeignete Vorrichtungen sein.

In weiterer besonderer Ausgestaltung des erfindungsgemäßen Reaktors umfasst dieser Mittel, die es gestatten, dass der aus der Vorwärmzone (a) abgeführte, abgekühlte Heißkondensatstrom der ersten Kühlzone (c1) in einer stromaufwärts angeordneten, vorgeschalteten Reaktionszelle als Kühlmedium zugeführt und anschließend zu dem Dampferzeuger zurückgeführt wird. Auf diese Weise wird die Wärmeintegration innerhalb des erfindungsgemäßen Reaktors verbessert und die Zu- und Abströme vom bzw. zum Dampferzeuger verringert.

Bevorzugt wird bei dem erfindungsgemäßen Reaktor mindestens ein Teil der Kühlzonen und/oder Vorwärmzonen als Plattenwärmetauscher mit Thermoblechen ausgestaltet. Die Thermobleche zeichnen sich dabei durch einen geringen Platzbedarf bei gleichzeitig guten Wärmeaustauscheigenschaften aus. Wenn auch die Reaktionszonen mit Thermoblechen ausgestattet sind, ergeben sich weitere Vorteile hinsichtlich einer konsistenteren Reaktorkonstruktion sowie logistische Vorteile für das Vorhalten von Ersatzteilen.

In einem weiteren Aspekt des erfindungsgemäßen Reaktors umfasst dieser Mittel, die es gestatten, einer oder mehrerer der stromabwärts der ersten Reaktionszelle angeordneten, nachgeschalteten Reaktionszellen frisches, noch nicht teilumgesetztes Einsatzgemisch zuzuführen. Alternativ oder zusätzlich können auch einzelne Reaktanten zugeführt werden, wie beispielsweise CO, CO₂ oder H₂ im Falle der Methanolsynthese, um die Kinetik oder Gleichgewichtslage der Reaktion gezielt zu beeinflussen. Mittel der genannten Art können Rohrleitungen, Regeleinrichtungen wie Ventile sowie andere für diesen Zweck geeignete Vorrichtungen sein. Vorteilhaft ist es dabei, dass das höhere chemische Reaktionspotential des noch nicht vorumgesetzten, frisch zugeführten Einsatzgemisch-Teilstroms genutzt wird, so dass die Gleichgewichtsreaktion in Richtung auf die Zielprodukte verschoben wird.

In weiterer besonderer Ausgestaltung des erfindungsgemäßen Reaktors ist die Reaktionszone (b) in mindestens einer Reaktionszelle mit mindestens zwei Katalysatoren ausgestattet, die hinsichtlich der exothermen Gleichgewichtsreaktion eine unterschiedliche Aktivität aufweisen. Die katalytische Aktivität der ersten, stromaufwärts angeordneten Katalysatorschicht kann beispielsweise höher sein als die katalytische Aktivität der zweiten, stromabwärts angeordneten Katalysatorschicht, wobei unter der Aktivität der Umsetzungsgrad im Katalysator pro Längeneinheit der Katalysatorschüttung verstanden wird.

Durch das Vorsehen einer ersten, stromaufwärtigen Schicht von Katalysatormaterial mit höherer katalytischer Aktivität wird erreicht, dass am Anfang des Katalysatorbettes ein hoher Umsatz von Synthesegas erreicht wird und entsprechend viel Wärme frei wird. Hierdurch wird eine für die Reaktion optimale Temperatur von ungefähr 250 °C erzielt. Die weitere Katalysatorschicht mit niedrigerer katalytischer Aktivität verhindert oder verringert die Ausbildung eines ausgeprägten Hot-Spots, der die Katalysatoraktivität rasch verschlechtern könnte. Ein sich ausbildender Hot-Spot wird mit steigender Betriebszeit von zunächst spitz und hoch zunehmend breiter und flacher und wandert zudem stromabwärts.

Vorteilhafterweise wird dabei die Schichtdicke der ersten stromaufwärtigen Katalysatorschicht deutlich geringer als die Schichtdicke der stromabwärtigen Schicht gewählt. In diesem Fall dient die stromaufwärtige, hochaktive Katalysatorschicht dem Aufheizen des Katalysatorbetts auf eine optimale Temperatur. Im weiteren Verlauf des Katalysatorbetts, d. h. im Bereich der zweiten stromabwärtigen Katalysatorschicht mit niedrigerer Aktivität, wird weniger Wärme frei und eine Desaktivierung des Katalysators wird verhindert. Die Schichtdicke der ersten Katalysatorschicht ist so gewählt, dass in dieser dünnen Schicht keine Temperatur erreicht wird, die die Ausbildung eines ausgeprägten Hot-Spots ermöglichen würde.

Eine weitere Ausgestaltungsmöglichkeit ergibt sich durch Verwendung unterschiedlicher Katalysatorformen in unterschiedlichen Reaktionszonen und/oder in unterschiedlichen Katalysatorschichten. Hierdurch wird der Wirkungsgrad pro Katalysatorpellet verbessert und dadurch der Umsatz zusätzlich erhöht.

Die Erfindung betrifft auch die Verwendung eines Reaktors gemäß einer der oben beschriebenen Ausgestaltungen zum Herstellen von Methanol durch Umsetzen eines Wasserstoff und Kohlenoxide, insbesondere Kohlendioxid, enthaltenden Synthesegaseinsatzes. Hierbei ist es besonders günstig, dass das Reaktionsprodukt Methanol durch technisch einfache Maßnahmen in den einzelnen Reaktionszellen auskondensiert werden kann.

### Ausführungsbeispiele

Weiterbildungen, Vorteile und Anwendungsmöglichkeiten der Erfindung ergeben sich auch aus der nachfolgenden Beschreibung von Ausführungsbeispielen und den Zeichnungen.

Es zeigen:
- Fig. 1: eine Reaktionszelle in einem Reaktor gemäß einer ersten Ausführungsform der Erfindung,
- Fig. 2: eine Reaktionszelle in einem Reaktor gemäß einer zweiten Ausführungsform der Erfindung,
- Fig. 3: ein erstes Beispiel für die Verschaltung zweier aufeinanderfolgender Reaktionszellen in einem erfindungsgemäßen Reaktor,
- Fig. 4: ein zweites Beispiel für die Verschaltung zweier aufeinanderfolgender Reaktionszellen in einem erfindungsgemäßen Reaktor,
- Fig. 5: ein Ausführungsbeispiel für die Verschaltung eine Reaktionszelle in einem erfindungsgemäßen Reaktor mit einem Dampferzeuger.

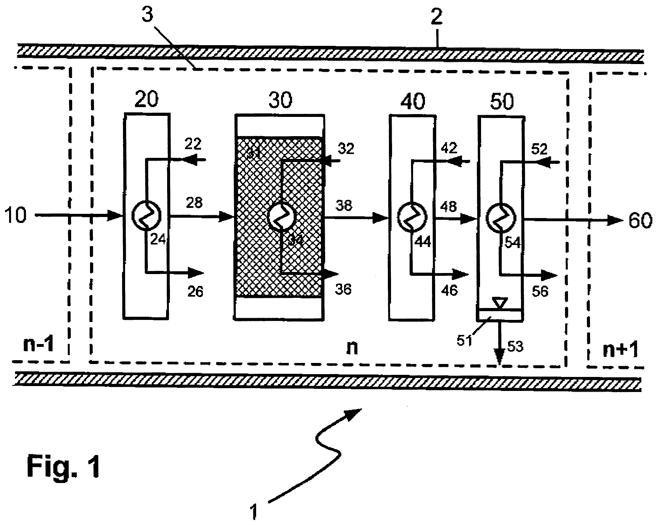

In Fig. 1 ist eine Reaktionszelle 3 in einem Reaktor 1 gemäß einer ersten Ausführungsform der Erfindung schematisch dargestellt. Die Reaktionszelle n befindet sich innerhalb des Reaktormantels 2, dessen Innenwand die physikalische Begrenzung des Reaktors nach außen bildet und den für die Durchführung der exothermen Gleichgewichtsreaktion gewählten Druck trägt.

Über Leitung 10 wird der Vorwärmzone 20 in der Reaktionszelle n der gasförmige, vorreagierte Produktstrom aus der vorgelagerten, stromaufwärts angeordneten Reaktionszelle n-1 zugeführt. Falls es sich bei der Reaktionszelle n um die erste Reaktionszelle in Strömungsrichtung handelt, wird über Leitung 10 das Einsatzgemisch zugeführt.

In der Vorwärmzone 20 wird der gasförmige Produktstrom oder das Einsatzgemisch auf die Reaktionstemperatur aufgeheizt. Dies erfolgt im indirekten Wärmetausch gegen ein Heizfluid, das über Leitung 22 dem Wärmetauscher 24 zugeführt wird und seinen Wärmeinhalt dort an den gasförmigen Produktstrom oder das Einsatzgemisch überträgt. Das abgekühlte Heizfluid wird über Leitung 26 aus dem Wärmetauscher abgeführt und in einer bildlich nicht dargestellten Heizvorrichtung aufgeheizt, um es erneut dem Wärmetauscher 24 zuzuführen.

Das aufgeheizte Einsatzgemisch bzw. der aufgeheizte, gasförmige Produktstrom wird über Leitung 28 der Reaktionszone 30 zugeführt, die eine Schüttung eines für die durchzuführende exotherme Gleichgewichtsreaktion aktiven Katalysators 31 und eine mit dem Katalysator in einer Wärmeaustauschbeziehung stehende Kühlvorrichtung 34 enthält. Die Abfuhr der durch die exotherme Reaktion freigesetzten Reaktionswärme erfolgt im indirekten Wärmetausch gegen ein Kühlfluid, welches optional teilweise verdampft, das über Leitung 32 dem Wärmetauscher 34 zugeführt und nach Aufnahme der in der Katalysatorschüttung freigesetzte Reaktionswärme über Leitung 36 abgeführt wird. Das aufgeheizte Kühlfluid wird in einer bildlich nicht dargestellten Kühlvorrichtung erneut abgekühlt, um es erneut dem Wärmetauscher 34 zuzuführen.

In der Reaktionszone erfolgt unter den gewählten Reaktionsbedingungen in der Katalysatorschüttung eine Teilumsetzung des Einsatzgemischs bzw. des gasförmigen Produktstroms aus der Reaktionszelle n-1 in einen mit kondensierbarem Reaktionsprodukt beladenen, gasförmigen Produktstrom, der über Leitung 38 aus der Reaktionszone abgeführt und einer ersten Kühlzone 40 zugeführt wird.

In der ersten Kühlzone 40 erfolgt eine Vorkühlung des mit kondensierbarem Reaktionsprodukt beladenen, gasförmigen Produktstroms, wobei bereits erste Anteile an Kondensat erhalten werden können, die über eine bildlich nicht dargestellte Abscheidevorrichtung und Leitungen aus dem Reaktor 1 ausgeleitet werden können. Alternativ kann die Vorkühlung in der ersten Kühlzone aber auch so durchgeführt werden, dass der Taupunkt des Gasstroms noch nicht unterschritten wird. Die Vorkühlung erfolgt im indirekten Wärmetausch gegen ein Kühlfluid, das über Leitung 42 dem Wärmetauscher 44 zugeführt und nach Wärmeaufnahme über Leitung 46 abgeführt wird. Das aufgeheizte Kühlfluid wird in einer bildlich nicht dargestellten Kühlvorrichtung erneut abgekühlt, um es erneut dem Wärmetauscher 44 zuzuführen.

Der vorgekühlte, aber immer noch mindestens mit einem Teil des kondensierbaren Reaktionsprodukts beladene, gasförmigen Produktstrom wird über Leitung 48 aus der ersten Kühlzone ausgeleitet und der zweiten Kühlzone 50 zugeführt. In der zweiten Kühlzone 50 erfolgt die weitere Abkühlung des mit kondensierbarem Reaktionsprodukt beladenen, gasförmigen Produktstroms, wobei dessen Taupunkt unterschritten wird. Hierbei wird ein flüssiges Kondensat erhalten, das mittels einer in die zweite Kühlzone integrierten Abscheidevorrichtung 51 vom Gasstrom abgetrennt und mittels Leitung 53 aus dem Reaktor ausgeleitet und der bildlich nicht dargestellten Produktaufarbeitung zugeführt wird. Die Abkühlung erfolgt im indirekten Wärmetausch gegen ein Kühlfluid, das über Leitung 52 dem Wärmetauscher 54 zugeführt und nach Wärmeaufnahme über Leitung 56 abgeführt wird. Das aufgeheizte Kühlfluid wird in einer bildlich nicht dargestellten Kühlvorrichtung erneut abgekühlt, um es erneut dem Wärmetauscher 54 zuzuführen.

Der abgekühlte und von Kondensat befreite, gasförmige Produktstrom wird über Leitung 60 aus der zweiten Kühlzone 50 und somit auch aus der Reaktionszelle n ausgeleitet. Er wird dann der stromabwärts angeordneten Reaktionszelle n+1 zugeführt, um eine weitere Umsetzung der gasförmigen Reaktanten zu Zielprodukten zu ermöglichen. Wenn keine weitere Umsetzung der gasförmigen Reaktanten erwünscht oder möglich ist, wird das verbliebene Restgas über Leitung 60 aus dem Reaktor ausgeleitet und der weiteren Aufarbeitung bzw. Entsorgung zugeführt. Alternativ kann der Restgasstrom nach Rückführen und Vermischen mit frischem Einsatzgemisch erneut dem Reaktor aufgegeben werden.

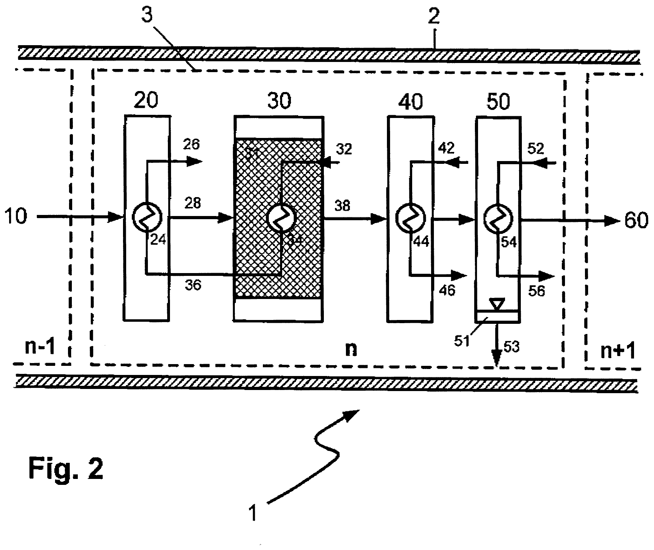

In den in Fig. 2 bis Fig. 5 schematisch dargestellten Ausgestaltungen des erfindungsgemäßen Reaktors entsprechen gleiche Bezugszeichen grundsätzlich den Vorrichtungsbestandteilen, wie sie bereits bei der Erläuterung der ersten Ausgestaltung der Erfindung, Fig. 1, beschrieben wurden. Auch die jeweiligen Arbeitsschritte und Verfahrensbedingungen sind gleich, sofern nicht nachfolgend anders beschrieben.

Im Unterschied zu der ersten Ausgestaltung wird in Fig. 2 das durch Aufnahme der Reaktionswärme in der Reaktionszone 30 aufgeheizte Kühlfluid über Leitung 36 zum Wärmetauscher 24 der Vorwärmzone 20 geführt und dort als Heizfluid für das Aufheizen des Einsatzgemischs oder des gasförmigen Produktstroms aus der vorgelagerten Reaktionszelle genutzt. Auf diese Weise wird die Wärmeintegration innerhalb des Reaktors verbessert. Diese Option ist besonders interessant, wenn man ein (teil-)verdampfendes Kühlmedium in der Reaktionszone 30 einsetzt und in der Vorwärmzone 20 zumindest teilweise wieder kondensiert und dort als Heizmedium nutzt. Bei einer vertikalen Anordnung von Vorwärmzone (oben) und darunter liegender Reaktionszone lässt sich dies einfach mit einer einzigen Anordnung, enthaltend eine obere Vorwärmzone ohne Katalysator und eine untere Reaktionszone mit Katalysator realisieren, die auf der Wärmeträgerseite direkt verbunden sind. Entstehender Dampf aus der Reaktionszone steigt auf und wird zumindest teilweise als Heizmedium in der Vorwärmzone genutzt, kondensierter Dampf fließt als Flüssigkeit zurück zur Reaktionszone. Das durch Wärmetausch mit dem in Leitung 10 zugeführten Gasstrom abgekühlte Heizfluid kann anschließend, optional nach weiterer Abkühlung in einer bildlich nicht dargestellten Kühlvorrichtung, als Kühlfluid über Leitung 32 zu dem Wärmetauscher 34 der Reaktionszone 30 zurückgeführt werden.

In einer weiteren, bildlich nicht gezeigten Ausgestaltung enthält die Reaktionszone zwei Schüttungen von Katalysatoren (erste und zweite Katalysatorschüttung) mit unterschiedlicher Aktivität für die exotherme Gleichgewichtsreaktion, die nacheinander von dem Einsatzgemisch bzw. gasförmigen Produktstrom aus der vorgelagerten Reaktionszelle durchströmt werden. In dieser Ausgestaltung wird nur die stromabwärts angeordnete Katalysatorschüttung mittels einer Kühlvorrichtung gekühlt. Eine Ausgestaltungsmöglichkeit sieht vor, dass die Katalysatorschüttung einen zweiten Katalysator enthält, der im Vergleich zu der ersten Katalysatorschüttung eine höhere Aktivität aufweist. Hierdurch kann die katalytische Umsetzung zunächst in Gang gesetzt werden und die dabei freigesetzte Wärmemenge trägt zu der Aufheizung des Reaktionsgemischs auf die gewählte Eintrittstemperatur in die zweite Katalysatorschüttung bei, wodurch der Wärmetauscher in der Vorwärmzone verkleinert werden kann. Für diese Funktion als Zündkatalysator reicht erfahrungsgemäß ein in Bezug auf die Haupt-Katalysatorschüttung (zweiter Katalysator) kleines bzw. kurzes Katalysatorbett aus. Die Reaktion in der Haupt-Katalysatorschüttung (zweiter Katalysator) verläuft dann gleichmäßiger, da Konzentrationsspitzen der Reaktanten bereits in der ersten Katalysatorschüttung reduziert werden und zudem die zweite Katalysatorschüttung gekühlt wird. Hierdurch wird die Bildung von Hot-Spots vermieden.

Alternativ kann in der ersten Katalysatorschüttung ein Katalysator eingesetzt werden, der im Vergleich zu der zweiten Katalysatorschüttung eine geringere Aktivität aufweist. Dies empfiehlt sich besonders dann, wenn das Reaktionspotential des in die Reaktionszone eintretenden Gasgemischs hoch ist. Dies ist in einer Ausgestaltung der Fall, in der der Reaktionszone frisches, also noch nicht vorreagiertes Einsatzgemisch oder einzelne Reaktanten zugeführt wird. Hierdurch wird die Reaktion langsamer und kontrollierter in Gang gesetzt und der Hauptteil der Reaktionswärme wird in der gekühlten zweiten Katalysatorschüttung freigesetzt.

Das Zuführen frischen, noch nicht vorreagierten Einsatzgemischs oder einzelner Reaktanten zu Reaktionszellen mit n > 1 kann auch in Verbindung mit den anderen hier erörterten Ausgestaltungen des erfindungsgemäßen Reaktors sinnvoll sein. Ferner kann es vorteilhaft sein, frisches, noch nicht vorreagiertes Einsatzgemisch zu mehr als einer Reaktionszelle mit n > 1 zuzuführen.

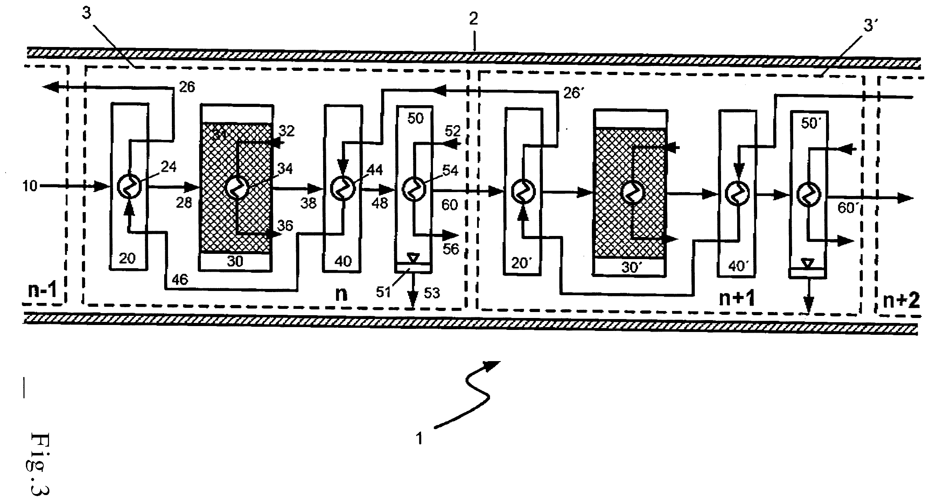

In der in Fig. 3 schematisch dargestellten Ausgestaltung des erfindungsgemäßen Reaktors ist eine Verschaltungsmöglichkeit zweier aufeinanderfolgender Reaktionszellen n und n+1 gezeigt. Entsprechende Vorrichtungsbestandteile der Reaktionszelle n+1 sind mit einem Apostroph an dem jeweiligen Bezugszeichen gekennzeichnet. Hierbei wird abgekühltes Heizfluid aus der Vorwärmzone 20' der Reaktionszelle n+1 über Leitung 26' dem Wärmetauscher 44 in der ersten Kühlzone 40 der Reaktionszelle n zugeführt und dient dort zur Vorkühlung des aus der Reaktionszone 30 über Leitung 38 abgeführten Gasstroms. Entsprechend wird abgekühltes Heizfluid aus der Vorwärmzone 20 der Reaktionszelle n über Leitung 26 dem entsprechenden Wärmetauscher in der ersten Kühlzone der Reaktionszelle n-1 zugeführt. Auf diese Weise wird eine noch weitergehende Wärmeintegration innerhalb des Reaktors erreicht, die sich nunmehr über mehrere Reaktionszellen erstreckt. Das im Wärmetauscher 44 aufgeheizte Heizfluid wird über Leitung 46 dem Wärmetauscher 24 zugeführt und dient dort zur Vorheizung des in die Reaktionszelle n über Leitung 10 eintretenden Gemischs.

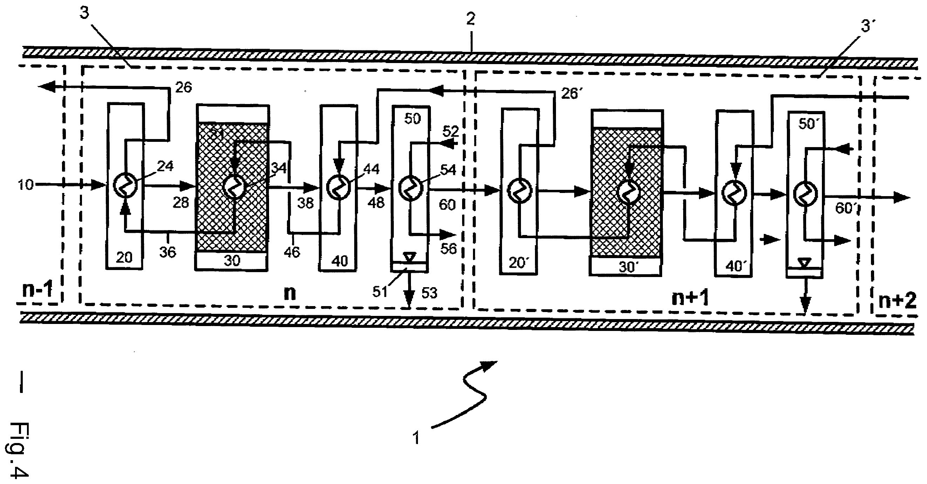

Im Unterschied zu der zuvor erörterten Ausgestaltung gemäß Fig. 3 wird in dem Ausführungsbeispiel der Fig. 4 zusätzlich das aus der jeweiligen ersten Kühlzone 40, 40' usw. abgeführte, aufgeheizte Kühlmittel dem Wärmetauscher der jeweils vorgeschalteten Reaktionszone 30, 30' usw. als Kühlmittel zugeführt. Das in der Reaktionszone weiter aufgeheizte Kühlmittel wird nachfolgend dem Wärmetauscher der jeweils vorgeschalteten Vorwärmzone als Heizfluid zugeführt. Diese Ausgestaltung kann sich insbesondere zur Durchführung exothermer Reaktionen mit mäßiger Wärmetönung eignen. Günstig ist im Rahmen dieser Ausgestaltung weiterhin die Verwendung eines Kühlfluids bzw. Heiz-fluids mit hoher Wärmeaufnahme- bzw. Wärmeabgabekapazität; hierfür eignen sich insbesondere Fluide, die bei ihrer Verwendung als Kühlfluid bzw. Heizfluid einen Phasenübergang flüssig - dampfförmig bzw. umgekehrt aufweisen. Schließlich kann es sinnvoll sein, die Reaktionszonen durch weitere, bildlich nicht dargestellte Kühlvorrichtungen zu kühlen, um eine intensivere Kühlwirkung und mehr Freiheitsgrade hinsichtlich der Temperaturführung in der Reaktionszone zu haben.

Bei den beiden zuletzt erörterten Ausgestaltungen des erfindungsgemäßen Reaktors kann es zudem sinnvoll sein, die aufgeheizten Kühlfluide bzw. abgekühlten Heizfluide zunächst zu einer oder mehrerer außerhalb des Reaktors angeordneten Kühl- bzw. Heizvorrichtungen zuzuführen, um die volle Wärmeaufnahme- bzw. Wärmeabgabekapazität des jeweiligen Fluids wiederherzustellen. Die Anordnung dieser externen Kühl- bzw. Heizvorrichtungen könnte beispielsweise im Leitungsweg der Leitungen 26, 26' usw. (Aufheizen), 46, 46' usw. (Kühlen) bzw. 36, 36' usw. (Kühlen) erfolgen.

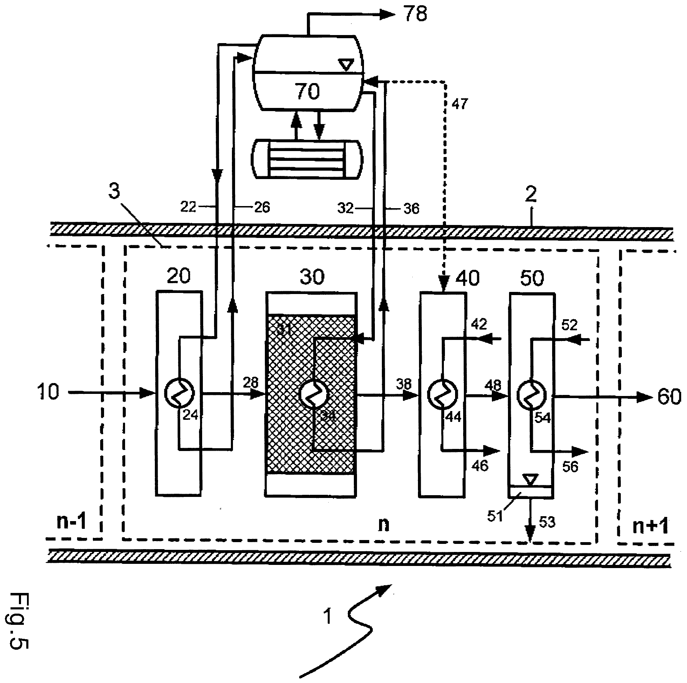

Die Verschaltung mit einer externen Kühl- bzw. Heizvorrichtung ist schematisch in der in Fig. 5 dargestellten Ausgestaltung gezeigt, bei der ein Dampferzeuger 70 außerhalb des Reaktors angeordnet ist. Diesem wird Heißkondensat entnommen und über Leitung 32 dem Wärmetauscher 34 der Reaktionszone 30 als Kühlmittel zugeführt, wobei es teilverdampft wird. Das resultierende Zweiphasengemisch flüssig - dampfförmig wird über Leitung 36 zum Dampferzeuger zurückgeführt.

Das Heißkondensat aus dem Dampferzeuger 70 kann ferner als Kühlmittel in der ersten Kühlzone 40 verwendet werden; dies ist schematisch durch die gestrichelte Leitung 47 dargestellt.

Dem Dampferzeuger 70 wird ferner Sattdampf entnommen, welcher über Leitung 22 dem Wärmetauscher 24 der Vorwärmzone 20 zugeführt wird. Durch die Wärmeabgabe an den über Leitung 10 herangeführten Strom erfolgt zumindest eine Teilkondensation. Der resultierende Strom kann entweder direkt über Leitung 26 zum Dampferzeuger zurückgeführt oder mittels anderer Vorrichtungen (bildlich nicht dargestellt) gesammelt werden und dann wieder zumindest teilweise zum Dampferzeuger zurückgeführt werden, um dort erneut verdampft zu werden.

In dem Ausführungsbeispiel der Fig. 5 kann ferner über eine Leitung 78 Sattdampf aus dem Dampferzeuger 70 abgeführt und als Exportdampf an externe Verbraucher abgegeben werden.

Als Wärmeträger bzw. Kühlmedium werden bevorzugt Medien eingesetzt, die sich in der Nähe ihres Siedepunktes befinden und daher leicht verdampfen (Kühlmedium) oder kondensieren (Wärmeträger, Heizmedium). Dies sichert eine gute Wärmeabfuhr durch den guten Wärmeübergang auf der Seite des verdampfenden bzw. kondensierenden Mediums und erlaubt eine präzise Temperaturregelung über den Druck. Um unterschiedliche Temperaturbedingungen in den verschiedenen Stufen einzustellen, wird der Druck auf der Seite des Wärmeträgers bzw. Kühlmediums individuell für jede Stufe geregelt. Mit zunehmender Katalysatorlaufzeit werden die Bedingungen mittels entsprechender Einstellung des Druckes auf der Kühlmediumseite angepasst und somit die Reaktionstemperatur nachgeführt, um den Umsatz entsprechend hoch zu halten.

Bezüglich der gewünschten Reaktionsbedingungen kann beispielsweise bei der Methanolsynthese Wasserdampf als Wärmeträger eingesetzt werden. Jedoch zeigt sich, dass bei Verwendung von Wasser für den gewünschten Temperaturbereich relativ große Druckunterschiede eingestellt werden müssen, um einen breiten Temperaturbereich abzudecken (z. B. 250 °C: ca. 40 bar, 264 °C: ca. 50 bar). Benutzt man dagegen ein verdampfendes Wärmeträgeröl (z. B. Dowtherm A) in einem Kreislauf zur Wasserdampferzeugung, so kann man in einem sehr engen Druckbereich arbeiten und dennoch einen großen Temperaturbereich abdecken (Bsp.: 255 °C: 0,97 bar, 305 °C: 2,60 bar, entsprechend einem Temperaturbereich von 50 °C bei einem Druckunterschied von nur 1,6 bar).

Dadurch kann man mit einer einfachen Wärmeträgeröl-Dampftrommel auf dem entsprechendem Anlagenniveau (ca. 20 bis 25 m) arbeiten und allein die Höhendifferenz nutzen, um die individuellen Druck- bzw. Temperaturbereiche einzustellen.

In den Abkühlzonen und/oder Kondensationszonen kann Kühlwasser oder auch ein verdampfender Wärmeträger eingesetzt werden, während in den Heizzonen ein kondensierender oder auch flüssiger Wärmeträger zum Einsatz kommen kann.

Bei vielen Ausgestaltungen des erfindungsgemäßen Reaktors, beispielsweise bei allen zuvor erörterten Ausgestaltungen, kann es vorteilhaft sein, die Wärmetransporträume durch jeweils wenigstens ein Thermoblech auszubilden. Unter den Wärmetransporträumen werden dabei die Bereiche des Reaktors verstanden, in denen es zum Wärmetausch zwischen der die Reaktanten bzw. Reaktionsprodukte enthaltenden Gasströmung und Heiz- bzw. Kühlfluiden kommt, also die Vorwärmzone, die Reaktionszone und die Kühlzonen.

Ein Thermoblech im Sinne der Erfindung besteht aus zwei Blechen, welche an den Rändern verbunden, vorzugsweise zusammengeschweißt sind und über deren Oberfläche eine Vielzahl von zusätzlichen Verbindungen, vorzugsweise Punktschweißungen, die die Platten ebenfalls miteinander verbinden, verteilt sind. Solche Platten können automatisiert von Robotern oder Maschinen und somit zu sehr günstigen Preisen hergestellt werden. Nach der Verschweißung werden die Bleche durch eine hydraulische Umformung, in der Regel das Einpressen einer Flüssigkeit unter hohem Druck, expandiert, wodurch kissenartige Kanäle zwischen den Blechen entstehen, durch die ein Heiz- oder Kühlfluid geleitet werden kann. Über die Wärmetransporträume kann daher bestimmten Bereichen des Reaktor durch das Durchleiten von Heiz- bzw. Kühlfluiden sowohl Wärmeenergie zu- als auch abgeführt werden.

Die Reaktionszonen können bei Verwendung von Thermoblechen so ausgestaltet werden, dass in dem Reaktor zunächst zwei Thermobleche im Wesentlichen parallel angeordnet werden. Im Wesentlichen parallel im Sinne der Erfindung bedeutet, dass die Ausrichtung der Thermobleche zueinander von der Parallelen maximal um +/- 20°, bevorzugt maximal um +/- 10°, besonders bevorzugt maximal um +/- 5°, ganz besonders bevorzugt maximal um +/- 2° voneinander abweicht. Danach kann der Freiraum zwischen den Thermoblechen mit einer Schüttung eines festen, körnigen, partikelförmigen oder pelletförmigen Katalysators aufgefüllt werden, wobei der seitliche Abschluss des sich ergebenden Katalysatorbettes durch Netze, Gitter, Lochbleche, Roste, Schüttungen von Inertmaterial und/oder die Reaktorinnenwand gebildet wird.

Besonders bevorzugt schließen sich parallel zu dieser Anordnung mindestens ein, bevorzugt mehrere weitere, beabstandete Thermobleche an, wobei sich insgesamt ein Plattenpaket ergibt und die Freiräume zwischen den Thermoblechen mit Katalysatorschüttungen aufgefüllt werden. Auf diese Weise wird in der Reaktionszone eine kompakte, sandwichartige Struktur mit einer intensiv wirkendenden, sich über die Länge der Reaktionszone erstreckenden Kühlvorrichtung erhalten. Die Beaufschlagung der einzelnen Katalysatorschüttungen mit dem Reaktionsgasgemisch erfolgt dabei parallel. Die Plattenpakete können, bezogen auf die mit Katalysator gefüllten Freiräume, parallel oder senkrecht zur Längsachse des Reaktors ausgerichtet werden.

Die Abstände zwischen den Thermoplatten werden gemäß der Wärmetönung der durchzuführenden Reaktion ausgewählt: Für stark exotherme Reaktionen wird dieser Abstand kleiner gewählt als für schwächer exotherme Reaktionen. Dabei werden in der ersten Reaktionszone kleinere Plattenabstände bevorzugt, da hier der größte Umsatz erzielt wird und die größte Wärmeabfuhr realisiert werden muss. Die Thermoblechplattenabstände der ersten Reaktionszone betragen im Falle der Methanolsynthese bevorzugt 20 bis 45 mm. Der Abstand bezieht sich dabei auf den Abstand von Mittellinie zur Mittellinie, d. h. der lichte Abstand zwischen den Platten fällt je nach Thermoblechdicke und Expansion des Hohlraumes entsprechend kleiner aus. Ferner wird der Abstand an die Abmessungen der Katalysatorpartikel angepasst, um eine optimale Wärmeabfuhr sowie ein gutes Schüttgutverhalten beim Befüllen und Entleeren des Katalysators ohne Brückenbildung sicherzustellen. In der zweiten und den nachfolgenden Reaktionszonen werden die Abstände üblicherweise größer gewählt.

Insbesondere bei horizontaler Anordnung des Reaktors bei gleichzeitig senkrechter Anordnung der Katalysatorschüttungen in den Reaktionszonen ergibt sich die Möglichkeit der einfachen Entfernung des Katalysators aus dem Reaktor zum Zwecke des Katalysatoraustauschs. Dabei sind zur Entleerung entsprechende Revisionsöffnungen im Reaktormantel vorzusehen, die beispielsweise durch einen Klapp- oder Schiebemechanismus betätigt werden. Der Schiebemechanismus kann sehr platzsparend ausgeführt werden, dabei ist es vorteilhaft, wenn sich die Tragroste der benachbarten Reaktionszonen mittels entsprechender Führungsschienen übereinander schieben lassen, so dass benachbarte Bereiche nacheinander entleert werden können.

In besonderer Ausgestaltung lassen sich sowohl stromabwärts als auch stromaufwärts der gekühlten Plattenpakete adiabate, also ungekühlte Reaktorbetten vorsehen. Das kann vor allem interessant werden, wenn nur noch ein Restumsatz erzielt werden soll und eine Kühlung der Reaktion aufgrund der geringen Wärmeentwicklung nicht mehr notwendig ist oder am Eintritt in eine Reaktionsstufe, wenn es vorteilhaft ist, eine schnelle Temperaturerhöhung zu erzielen, bevor die Reaktanten den gekühlten Bereich der Reaktionszone eintreten.

Auch bei der Ausgestaltung der Vorwärmzone und der Kühlzonen können Thermobleche vorteilhaft im Sinne eines Plattenwärmetauschers eingesetzt werden. Auf die Verwendung von Rohrendplatten, wie sie bei Rohrbündelwärmetauschern erforderlich sind, kann dabei verzichtet werden. Zudem werden logistische und fertigungstechnische Vorteile erhalten, da sich die Zahl unterschiedlicher Bauteile des Reaktors und damit die Komplexität des Apparates reduzieren.

Eine weitere Ausgestaltungsmöglichkeit des erfindungsgemäßen Reaktors wird durch die Ausgestaltung der Wärmetransporträume mittels Lamellenwärmetauschern (plate fin heat exchanger) alternativ oder zusätzlich zu der Verwendung von Thermoblechen ermöglicht.

### Zahlenbeispiele

### Vergleich des erfindungsgemäßen Reaktors mit aus dem Stand der Technik bekannten Reaktoren

In den nachfolgenden Tabellen werden Kenndaten des erfindungsgemäßen Reaktors mit aus dem Stand der Technik bekannten Reaktoren für die heterogen-katalytische Synthese von Methanol aus Synthesegas verglichen.

Im ersten Vergleichsfall wird ein erfindungsgemäßer Reaktor mit drei Reaktionszellen einem dreistufigen technischen Reaktor gegenübergestellt, der zwei parallel geschaltete wassergekühlte Reaktoren WCR, stromabwärts gefolgt von einem gasgekühlten Reaktor GCR, umfasst. Die technische Anlage verfügt über keine Zwischenkondensation zwischen WCR und GCR. Das Einsatzgas ist in beiden Fällen hinsichtlich Zusammensetzung und Stoffmengenstrom gleich, es handelt sich um ein Synthesegas mit folgender Zusammensetzung: 8,4 Vol.-% CO₂, 20,1 Vol.-% CO, 68 Vol.-% H₂, Rest Inertkomponenten. Der Eintrittsdruck in den Reaktor beträgt jeweils 75 bar, Überdruck. In Tabelle 1 sind die wesentlichen Vergleichsdaten für beide Reaktoren zusammengestellt. Dabei bedeutet Xₚₚ(k) den Umsatz der Komponente k pro Durchgang durch den Reaktor (per pass) und Xtot(k) ihren Gesamtumsatz über den Reaktor inklusive Gaskreislauf. STY ist die Raum-Zeit-Ausbeute an Methanol in kg/h, bezogen auf einen Liter Katalysatorvolumen.

**Tabelle 1: Vergleich der Kenndaten des erfindungsgemäßen Reaktors mit drei Reaktionszellen mit einem dreistufigen Methanolsynthesereaktor (2 WCR parallel + GCR) gemäß Stand der Technik.**

| | **Methanolsynthesereaktor mit 2 WCR parallel + GCR Vergleichsbeispiel** | **Erfindungsgemäßer Reaktor (drei Reaktionszellen)** |
|---|---|---|
| Xₚₚ(CO) / % | 81,9 | 95,5 |
| Xₚₚ(CO₂) / % | 28,0 | 60,7 |
| Xₚₚ(COₓ) / % | 54,6 | 82,7 |
| Xₜₒₜ(CO) / % | 99,2 | 99,1 |
| Xₜₒₜ(CO₂) / % | 85,4 | 84,4 |
| Xₜₒₜ(COₓ) / % | 95,2 | 94,4 |
| STY(MeOH) / kg/(h Liter_{Kat}) | 0,86 | 1,26 |
| V_{Kat,tot}/ m³ | 315 | 180 |
| Tᵢₙ / °C | 230 | 215 |
| Tₘₐₓ / °C | 270 | 230 |
| Nebenprodukte / ppm | 6200 | 3250 |
| Rückführverhältnis | 2,2 | 0,5 |

Wie anhand der in Tabelle 1 zusammengestellten Daten zu erkennen ist, ist der Umsatz an Kohlenoxiden für den Gesamtreaktor in beiden Fällen vergleichbar; jedoch sind die Umsätze pro Reaktordurchgang für den erfindungsgemäßen Reaktor deutlich höher. Für letzteren sind zudem die Maximaltemperatur im Katalysatorbett, die Konzentration an Nebenprodukten und das benötigte Rückführverhältnis geringer.

In Tabelle 2 wird nachfolgend ein einstufiger, wassergekühlter Reaktor zur Methanolsynthese mit einem erfindungsgemäßen Reaktor verglichen, der vier Reaktionszellen umfasst, wobei der erfindungsgemäße Reaktor ohne Rückführung betrieben wird. Das Einsatzgas ist in beiden Fällen hinsichtlich Zusammensetzung und Stoffmengenstrom gleich, es handelt sich um ein Synthesegas mit folgender Zusammensetzung: 7 Vol.-% CO₂, 16 Vol.-% CO, 73 Vol.-% H₂, Rest Inertkomponenten. Der Eintrittsdruck in den Reaktor beträgt jeweils 75 bar, Überdruck.

**Tabelle 2: Vergleich der Kenndaten des erfindungsgemäßen Reaktors mit vier Reaktionszellen ohne Rückführung mit einem einstufigen wassergekühlten Methanolsynthesereaktor**

| | **Methanolsynthesereaktor (einstufiger gekühlter Reaktor mit hoher Gasrückführungsrate) Vergleichsbeispiel** | **Erfindungsgemäßer Reaktor (vier Reaktionszellen ohne Gasrückführung)** |
|---|---|---|
| Xₚₚ(CO) / % | 90,8 | 99,7 |
| Xₚₚ(CO₂) / % | 62,8 | 93,9 |
| Xₚₚ(COₓ) / % | 80,6 | 97,8 |
| Xₜₒₜ(CO) / % | 99,2 | 99,7 |
| Xₜₒₜ(CO₂) / % | 94,7 | 93,9 |
| Xₜₒₜ(COₓ) / % | 97,9 | 97,8 |
| STY(MeOH) / kg/(h Liter_{Kat}) | 0,98 | 1,15 |
| **Rückführverhältnis** | **3,5** | **0** |

Mit dem erfindungsgemäßen Reaktor mit vier Reaktionszellen wird ohne Rückführung eine um rund 15 % höhere Raum-Zeit-Ausbeute an Methanol erhalten. Insbesondere der CO₂-Umsatz pro Reaktordurchgang ist deutlich höher als bei dem Vergleichsbeispiel.

### Optimierung der Verfahrensbedingungen bei dem erfindungsgemäßen Reaktor

In den nachfolgenden Tabellen wird der Einfluss bestimmter Verfahrensparameter in den einzelnen Reaktionszellen des erfindungsgemäßen Reaktors bei der heterogen-katalytischen Synthese von Methanol aus Synthesegas aufgezeigt. Die sonstigen Verfahrensbedingungen entsprechen denen des in Tabelle 2 dargestellten Beispiels (in den Tabellen 3 bis 5 als Referenz bezeichnet).

**Tabelle 3: Variation der Verteilung des Katalysatorvolumens**

| **Fall** | **V_{Kat} / m³** | | | | | **Xₜₒₜ(COₓ) / %** | **STY(MeOH) / kg/(h I_{Kat})** |
|---|---|---|---|---|---|---|---|
| | 1.Stufe | 2.Stufe | 3.Stufe | 4.Stufe | gesamt | gesamt | gesamt |
| Referenz | 8 | 8 | 8 | 8 | 32 | 95,1 | 1,53 |
| 1 | 4 | 6 | 10 | 12 | 32 | 92,1 | 1,49 |
| 2 | 12 | 10 | 6 | 4 | 32 | 96 | 1,56 |

**Tabelle 4: Variation der Kühltemperatur Tcooi im Katalysatorbett**

| **Fall** | **T_{cool} / °C** | | | | Xₜₒₜ(COₓ) **/ %** | **STY(MeOH) / kg/(h I_{Kat})** |
|---|---|---|---|---|---|---|
| | 1. Stufe | 2. Stufe | 3. Stufe | 4. Stufe | gesamt | gesamt |
| Referenz | 220 | 220 | 220 | 220 | 95,1 | 1,53 |
| 3 | 200 | 220 | 240 | 260 | 89,4 | 1,44 |
| 4 | 260 | 240 | 220 | 200 | 96 | 1,55 |

**Tabelle 5: Variation der Kondensationstemperatur T_{cond}**

| **Fall** | **T_{cond} / °C** | | | | **Kühlleistung** / **MW** | **CO₂-Verluste / %** |
|---|---|---|---|---|---|---|
| | 1. Stufe | 2. Stufe | 3. Stufe | 4. Stufe | gesamt | gesamt |
| Referenz | 40 | 40 | 40 | 40 | 39,9 | 7,4 |
| 5 | 100 | 80 | 60 | 40 | 33,7 | 3,7 |
| 6 | 40 | 60 | 80 | 100 | 34,9 | 6,0 |

### Gewerbliche Anwendbarkeit

Mit der Erfindung wird ein Reaktor zum Durchführen exothermer Gleichgewichtsreaktionen, insbesondere für die Durchführung der Methanolsynthese durch heterogen-katalysierte Umsetzung von Synthesegas vorgeschlagen, der es ermöglicht, die Reaktionsbedingungen entlang der Längskoordinate des Reaktors nachzuführen und somit zu optimieren, was beispielsweise im Falle der Methanolsynthese zu einer Reduzierung des Rückführverhältnis auf kleinere Werte führt, wie sie bei Verwendung der aus dem Stand der Technik bekannten Reaktoren bekannt sind. Entsprechende Rückführleitungen, Kreislaufverdichter usw. können daher kleiner ausgestaltet werden oder es kann gegebenenfalls ganz auf sie verzichtet werden. Hierdurch reduzieren sich die entsprechenden Investitionskosten.

Durch die Optimierung der Reaktionsbedingungen entlang der Längskoordinate des Reaktors wird ferner die Bildung unerwünschter Nebenprodukte reduziert, wodurch ein reineres Zielprodukt erhalten wird und sich der Reinigungsaufwand verringert.

### Bezugszeichenliste

- [1]: Reaktor
- [2]: Reaktormantel
- [3]: Reaktionszelle
- [10]: Leitung
- [20]: Vorwärmzone
- [22]: Leitung
- [24]: Wärmetauscher
- [26]: Leitung
- [28]: Leitung
- [30]: Reaktionszone
- [31]: Katalysatorbett
- [32]: Leitung
- [34]: Wärmetauscher
- [35]: Leitung
- [36]: Leitung
- [38]: Leitung
- [40]: erste Kühlzone
- [42]: Leitung
- [44]: Wärmetauscher
- [46]: Leitung
- [47]: Leitung
- [48]: Leitung
- [50]: zweite Kühl- und Abscheidezone
- [51]: Abscheidevorrichtung
- [52]: Leitung
- [53]: Leitung
- [54]: Wärmetauscher
- [56]: Leitung
- [60]: Leitung
- [70]: Dampferzeuger
- [78]: Leitung

## Patentansprüche

1. Reaktor zum Durchführen exothermer Gleichgewichtsreaktionen, bei denen ein gasförmiges Einsatzgemisch an einem festen Katalysator mindestens teilweise zu einem Produktgemisch umgesetzt wird, das mindestens ein bei dem Reaktordruck und bei Temperaturen unterhalb der Reaktortemperatur kondensierbares, flüssiges Reaktionsprodukt enthält, umfassend mindestens zwei hintereinander geschaltete und in Fluidverbindung miteinander stehende Reaktionszellen, die in einem gemeinsamen Reaktormantel angeordnet sind, wobei jede Reaktionszelle die folgenden, hintereinander geschalteten und in Fluidverbindung miteinander stehenden Baugruppen umfasst:
(a) eine Vorwärmzone, geeignet zum Aufheizen des Einsatzgemischs oder des gasförmigen Produktstroms aus der vorgelagerten Reaktionszelle, wobei die Vorwärmzone in der ersten Reaktionszelle in Strömungsrichtung des gasförmigen Einsatzgemischs optional entfallen kann,
(b) mindestens eine Reaktionszone, enthaltend einen für die durchzuführende exotherme Gleichgewichtsreaktion aktiven Katalysator als Katalysatorschüttung in einem Katalysatorbett und eine mit dem Katalysatorbett in einer Wärmeaustauschbeziehung stehende Kühlvorrichtung,
(c) mindestens eine Kühlzone, enthaltend eine Kühlvorrichtung, geeignet zur Abkühlung des aus der Reaktionszone austretenden, teilumgesetzten und mit kondensierbarem Reaktionsprodukt beladenen, gasförmigen Produktstroms auf eine Temperatur unterhalb des Taupunktes dieses Gases,
(d) eine Abscheidezone, enthaltend eine Phasentrennvorrichtung zum Auftrennen des aus der Kühlzone austretenden Produktstroms in einen von Kondensat befreiten, gasförmigen Produktstrom und einen flüssiges Reaktionsprodukt umfassenden Kondensatstrom,
(e) Mittel zum Ausleiten des flüssiges Reaktionsprodukt umfassenden Kondensatstroms und optional Mittel zum Zuführen des Kondensatstroms zu einer Aufarbeitungsvorrichtung für das Reaktionsprodukt,
(f) Mittel zum Ausleiten des von Kondensat befreiten, gasförmigen Produktstroms und Mittel zum Zuführen dieses gasförmigen Produktstroms zu einer nachgelagerten, stromabwärts angeordneten Reaktionszelle und/oder Mittel zum Ausleiten des gasförmigen Produktstroms aus dem Reaktor.

2. Reaktor nach Anspruch 1, **dadurch gekennzeichnet, dass** die Kühlzone in Baugruppe (c) folgendes umfasst:
(c1) eine erste Kühlzone, enthaltend eine Kühlvorrichtung, geeignet zur Abkühlung des aus der Reaktionszone austretenden, teilumgesetzten und mit kondensierbarem Reaktionsprodukt beladenen, gasförmigen Produktstroms auf eine Temperatur unterhalb der Temperatur in der Reaktionszone,
(c2) eine zweite Kühlzone, enthaltend eine Kühlvorrichtung, geeignet zur weiteren Abkühlung des aus der ersten Kühlzone austretenden, teilumgesetzten und mit kondensierbarem Reaktionsprodukt beladenen, vorgekühlten gasförmigen Produktstroms auf eine Temperatur unterhalb des Taupunktes dieses Gases.

3. Reaktor nach Anspruch 1 oder 2, **dadurch gekennzeichnet, dass** der Mantel horizontal oder vertikal gegenüber der durch die Schwerkraft vermittelten Senkrechten angeordnet ist, wobei die Reaktionszellen in beiden Fällen senkrecht von dem gasförmigen Einsatzgemisch oder dem gasförmigen Produktstrom aus der vorgelagerten Reaktionszelle durchströmt werden.

4. Reaktor nach Anspruch 1 oder 2, **dadurch gekennzeichnet, dass** der Mantel horizontal oder vertikal gegenüber der durch die Schwerkraft vermittelten Senkrechten angeordnet ist, wobei die Reaktionszellen in beiden Fällen horizontal von dem gasförmigen Einsatzgemisch oder dem gasförmigen Produktstrom aus der vorgelagerten Reaktionszelle durchströmt werden.

5. Reaktor nach Anspruch 4, **dadurch gekennzeichnet, dass** der Mantel vertikal gegenüber der durch die Schwerkraft vermittelten Senkrechten angeordnet ist, wobei die Reaktionszellen horizontal und in radialer Richtung von dem gasförmigen Einsatzgemisch oder dem gasförmigen Produktstrom aus der vorgelagerten Reaktionszelle durchströmt werden.

6. Reaktor nach einem der vorigen Ansprüche, **dadurch gekennzeichnet, dass** die Reaktionszone (b) mit Thermoblechen ausgestattet ist, wobei die Thermobleche aus jeweils zwei miteinander verbundenen Blechen bestehen, wobei dieser Verbund auf seiner Innenseite einen gegenüber der Außenseite dicht abschließenden Hohlraum aufweist, der von einem fluiden Kühlmedium durchströmt wird, wobei der Katalysator in der Reaktionszone stückig oder partikelförmig als Festkörper-Schüttung vorliegt, die jeweils so zwischen zwei benachbarten Thermoblechen angeordnet ist, dass sie senkrecht oder horizontal von dem gasförmigen Einsatzgemisch oder dem gasförmigen Produktstrom aus der vorgelagerten Reaktionszelle durchströmt werden kann und wobei der Katalysator und das Kühlmedium in einer indirekten Wärmeaustauschbeziehung stehen.

7. Reaktor nach einem der vorigen Ansprüche, **dadurch gekennzeichnet, dass** die Vorwärmzone, die Reaktionszone oder die Kühlzonen oder mehrere dieser Baugruppen als Lamellenwärmetauscher ausgeführt werden.

8. Reaktor nach einem der vorigen Ansprüche, **dadurch gekennzeichnet, dass** als Kühlmedium Heißkondensat aus einem Dampferzeuger verwendet wird, wobei das Kühlmedium mindestens einen Teil der in der Reaktionszone (b) freigesetzten Reaktionswärme aufnimmt und dabei teilverdampft wird und wobei das dabei erhaltene Kondensat-Sattdampf-Gemisch oder der Sattdampf mindestens teilweise zu dem Dampferzeuger zurückgeführt wird und/oder als Wärmeträger zu der Vorwärmzone (a) derselben Reaktionszelle geführt wird.

9. Reaktor nach einem der vorigen Ansprüche, **dadurch gekennzeichnet, dass** Mittel umfasst werden, die es gestatten, dass das aus einer oder mehreren Reaktionszellen abgeführte Kondensat-Sattdampf-Gemisch oder nur der Dampfanteil mindestens teilweise zu dem Dampferzeuger zurückgeführt wird und der aus dem Dampferzeuger abgezogene Sattdampf mindestens zum Teil als Exportdampf an externe Verbraucher abgegeben wird.

10. Reaktor nach einem der vorigen Ansprüche, **dadurch gekennzeichnet, dass** Mittel umfasst werden, die es gestatten, dass in der Vorwärmzone (a) das Aufheizen des Einsatzgemischs oder des gasförmigen Produktstroms aus der vorgelagerten Reaktionszelle im indirekten Wärmeaustausch gegen Heißkondensat aus einem Dampferzeuger erfolgt, wobei ein abgekühlter Heißkondensatstrom erhalten wird.

11. Reaktor nach einem der vorigen Ansprüche, **dadurch gekennzeichnet, dass** Mittel umfasst werden, die es gestatten, dass der aus der Vorwärmzone (a) abgeführte, abgekühlte Heißkondensatstrom der ersten Kühlzone (c1) in einer stromaufwärts angeordneten, vorgeschalteten Reaktionszelle als Kühlmedium zugeführt und anschließend zu dem Dampferzeuger zurückgeführt wird.

12. Reaktor nach einem der vorigen Ansprüche, **dadurch gekennzeichnet, dass** mindestens ein Teil der Kühlzonen und/oder Vorwärmzonen als Plattenwärmetauscher mit Thermoblechen ausgestaltet sind.

13. Reaktor nach einem der vorigen Ansprüche, **dadurch gekennzeichnet, dass** Mittel umfasst werden, die es gestatten, einer oder mehrerer der stromabwärts der ersten Reaktionszelle angeordneten, nachgeschalteten Reaktionszellen frisches, noch nicht teilumgesetztes Einsatzgemisch oder einzelne Reaktanten zuzuführen.

14. Reaktor nach einem der vorigen Ansprüche, **dadurch gekennzeichnet, dass** die Reaktionszone (b) in mindestens einer Reaktionszelle mit mindestens zwei Katalysatoren ausgestattet ist, die hinsichtlich der exothermen Gleichgewichtsreaktion eine unterschiedliche Aktivität aufweisen.

15. Verwendung eines Reaktors gemäß Anspruch 1 bis 14 zum Herstellen von Methanol durch Umsetzen eines Kohlendioxid enthaltenden Synthesegaseinsatzes.

## Claims

1. Reactor for conducting exothermic equilibrium reactions, in which a gaseous feed mixture is at least partly converted over a solid catalyst to a product mixture comprising at least one liquid reaction product condensable at the reactor pressure and at temperatures below the reactor temperature, comprising at least two series-connected reaction cells that are in fluid connection with one another and are arranged in a common reactor shell, wherein each reaction cell comprises the following series-connected assemblies that are in fluid connection with one another:
(a) a preheating zone suitable for heating the feed mixture or the gaseous product stream from the upstream reaction cell, wherein the preheating zone can optionally be dispensed with in the first reaction cell in flow direction of the gaseous feed mixture,
(b) at least one reaction zone comprising a catalyst active in respect of the exothermic equilibrium reaction to be conducted as catalyst fill in a catalyst bed and a cooling apparatus in a heat-exchanging relationship with the catalyst bed,
(c) at least one cooling zone comprising a cooling apparatus suitable for cooling the partly converted, gaseous product stream that has been laden with condensable reaction product and exits from the reaction zone to a temperature below the dew point of this gas,
(d) a deposition zone comprising a phase separation apparatus for separation of the product stream that exits from the cooling zone into a gaseous product stream that has been freed of condensate and a condensate stream comprising liquid reaction product,
(e) means of discharging the condensate stream comprising liquid reaction product and optionally means of feeding the condensate stream to a workup apparatus for the reaction product,
(f) means of discharging the gaseous product stream that has been freed of condensate and means of feeding this gaseous product stream to a subsequent reaction cell arranged downstream and/or means of discharging the gaseous product stream from the reactor.

2. Reactor according to Claim 1, **characterized in that** the cooling zone comprises the following in assembly (c) :
(c1) a first cooling zone comprising a cooling apparatus suitable for cooling the partly converted, gaseous product stream that has been laden with condensable reaction product and exits from the reaction zone to a temperature below the temperature in the reaction zone,
(c2) a second cooling zone comprising a cooling apparatus suitable for further cooling the partly converted, precooled gaseous product stream that has been laden with condensable reaction product and exits from the first cooling zone to a temperature below the dew point of this gas.

3. Reactor according to Claim 1 or 2, **characterized in that** the shell is arranged horizontally or vertically with respect to the perpendicular imparted by gravity, wherein the flow of the gaseous feed mixture or the gaseous product stream from the upstream reaction cell through the reaction cells in both cases is vertical.

4. Reactor according to Claim 1 or 2, **characterized in that** the shell is arranged horizontally or vertically with respect to the perpendicular imparted by gravity, wherein the flow of the gaseous feed mixture or the gaseous product stream from the upstream reaction cell through the reaction cells in both cases is horizontal.

5. Reactor according to Claim 4, **characterized in that** the shell is arranged vertically with respect to the perpendicular imparted by gravity, wherein the flow of the gaseous feed mixture or the gaseous product stream from the upstream reaction cell through the reaction cells is horizontal and in radial direction.

6. Reactor according to any of the preceding claims, **characterized in that** the reaction zone (b) has been equipped with thermoplates, wherein the thermoplates consist of two sheets each bonded to one another, wherein this composite has, on its inside, a cavity which is tightly sealed from the outside and through which a fluid cooling medium flows, wherein the catalyst is present in the reaction zone in piece form or particulate form as a bed of solid material arranged between two adjacent thermoplates in each case in such a way that the gaseous feed mixture or the gaseous product stream from the upstream reaction cell can flow through it vertically or horizontally, and wherein the catalyst and the cooling medium are in an indirect heat-exchanging relationship.

7. Reactor according to any of the preceding claims, **characterized in that** the preheating zone, the reaction zone or the cooling zones or two or more of these assemblies are executed in the form of a lamellar heat exchanger.

8. Reactor according to any of the preceding claims, **characterized in that** the cooling medium used is hot condensate from a steam generator, wherein the cooling medium takes up at least a portion of the heat of reaction released in the reaction zone (b) and is partly evaporated, and wherein the condensate/saturated steam mixture obtained or the saturated steam is at least partly recycled to the steam generator and/or conducted as heat carrier to the preheating zone (a) of the same reaction cell.

9. Reactor according to any of the preceding claims, **characterized in that** means are encompassed which permit at least partial recycling of the condensate/saturated steam mixture removed from one or more reaction cells or of the steam component only to the steam generator and at least partial release of the saturated steam drawn off from the steam generator as export steam to external consumers.

10. Reactor according to any of the preceding claims, **characterized in that** means are encompassed which permit, in the preheating zone (a), the heating of the feed mixture or of the gaseous product stream from the upstream reaction cell in indirect heat exchange against hot condensate from a steam generator, to obtain a cooled hot condensate stream.

11. Reactor according to any of the preceding claims, **characterized in that** means are encompassed which permit supply of the cooled hot condensate stream from the first cooling zone (c1), removed from the preheating zone (a), as cooling medium in a preceding reaction cell arranged upstream, followed by recycling thereof to the steam generator.

12. Reactor according to any of the preceding claims, **characterized in that** at least some of the cooling zones and/or preheating zones are configured as plate heat exchangers with thermoplates.

13. Reactor according to any of the preceding claims, **characterized in that** means are encompassed which permit supply of fresh feed mixture that has not yet been partly converted or individual reactants to one or more of the subsequent reaction cells arranged downstream of the first reaction cell.

14. Reactor according to any of the preceding claims, **characterized in that** the reaction zone (b) is equipped, in at least one reaction cell, with at least two catalysts having different activity with regard to the exothermic equilibrium reaction.

15. Use of a reactor according to Claims 1 to 14 for preparation of methanol by conversion of a carbon dioxide-containing synthesis gas feed.

## Revendications

1. Réacteur destiné à effectuer des réactions d'équilibre exothermique dans lesquelles un mélange d'alimentation gazeux est au moins partiellement converti sur un catalyseur solide en un mélange de produits qui contient au moins un produit de réaction liquide condensable à la pression du réacteur et à des températures inférieures à la température du réacteur, ledit réacteur comprenant au moins deux cellules de réaction qui sont montées en série l'une derrière l'autre et reliées fluidiquement l'une à l'autre et qui sont disposées dans une enveloppe de réacteur commune, chaque cellule de réaction comprenant les composants suivants montés en série les uns derrière es autres et reliés fluidiquement les uns aux autres :
(a) une zone de préchauffage appropriée pour chauffer le mélange d'alimentation ou le flux de produit gazeux provenant de la cellule de réaction en amont, la zone de préchauffage dans la première cellule de réaction dans le sens d'écoulement du mélange d'alimentation gazeux pouvant éventuellement être supprimée,
(b) au moins une zone de réaction contenant un catalyseur, actif pour la réaction d'équilibre exothermique à effectuer, comme dépôt de catalyseur dans un lit de catalyseur et un dispositif de refroidissement qui est relié au lit de catalyseur dans une relation d'échange thermique,
(c) au moins une zone de refroidissement contenant un dispositif de refroidissement adapté pour refroidir le flux de produit gazeux, sortant de la zone de réaction, partiellement converti et chargé en produit de réaction condensable, à une température inférieure au point de rosée de ce gaz,
(d) une zone de séparation contenant un dispositif de séparation de phases destiné à séparer le flux de produit, sortant de la zone de refroidissement, en un flux de produit gazeux débarrassé de condensat et un flux de condensat comprenant un produit de réaction liquide,
(e) des moyens d'évacuation du flux de condensat, comprenant un produit de réaction liquide, et éventuellement des moyens d'amenée du courant de condensat à un dispositif de traitement du produit de réaction,
(f) des moyens d'évacuation du flux de produit gazeux, débarrassé de condensat, et des moyens d'amenée de ce flux de produit gazeux à une cellule de réaction montée en aval et disposée en aval du flux et/ou des moyens d'évacuation du flux de produit gazeux du réacteur.

2. Réacteur selon la revendication 1, **caractérisé en ce que** la zone de refroidissement du composant (c) comprend :
(c1) une première zone de refroidissement contenant un dispositif de refroidissement adapté pour refroidir le flux de produit gazeux sortant de la zone de réaction,
partiellement converti et chargé en produit de réaction condensable, à une température inférieure à la température dans la zone de réaction,
(c2) une deuxième zone de refroidissement contenant un dispositif de refroidissement approprié pour refroidir davantage le flux de produit gazeux pré-refroidi, sortant de la première zone de refroidissement, partiellement converti et chargé de produit de réaction condensable, à une température inférieure au point de rosée de ce gaz.

3. Réacteur selon la revendication 1 ou 2, **caractérisé en ce que** l'enveloppe est disposée horizontalement ou verticalement par rapport à la perpendiculaire fournie par la force de gravité, le mélange d'alimentation gazeux ou le flux de produit gazeux provenant de la cellule de réaction amont s'écoulant dans les deux cas verticalement à travers les cellules de réaction.

4. Réacteur selon la revendication 1 ou 2, **caractérisé en ce que** l'enveloppe est disposée horizontalement ou verticalement par rapport à la perpendiculaire fournie par la force de gravité, le mélange d'alimentation gazeux ou le flux de produit gazeux provenant de la cellule de réaction en amont s'écoulant dans les deux cas horizontalement à travers les cellules de réaction.

5. Réacteur selon la revendication 4, **caractérisé en ce que** l'enveloppe est disposée verticalement par rapport à la perpendiculaire fournie par la force de gravité, le mélange d'alimentation gazeux ou le flux de produit gazeux provenant de la cellule de réaction en amont s'écoulant horizontalement et dans la direction radiale à travers les cellules de réaction.

6. Réacteur selon l'une des revendications précédentes, **caractérisé en ce que** la zone de réaction (b) est équipée de tôles thermiques, les tôles thermiques comprenant chacune deux tôles reliées l'une à l'autre, ce composite comportant sur son côté intérieur une cavité qui est isolée de manière étanche du côté extérieur et à travers laquelle s'écoule un milieu de refroidissement fluide, le catalyseur étant présent dans la zone de réaction en morceaux ou sous forme de particules comme dépôt solide qui est disposé entre deux tôles thermiques adjacentes de sorte que le mélange d'alimentation gazeux ou le flux de produit gazeux provenant de la cellule de réaction amont puisse s'écouler verticalement ou horizontalement, et le catalyseur et le milieu de refroidissement étant reliés par une relation d'échange thermique indirecte.

7. Réacteur selon l'une des revendications précédentes, **caractérisé en ce que** la zone de préchauffage, la zone de réaction ou les zones de refroidissement ou plusieurs de ces composants sont réalisés sous forme d'échangeurs de chaleur lamellaires.

8. Réacteur selon l'une des revendications précédentes, **caractérisé en ce qu'**on utilise comme milieu de refroidissement un condensat chaud d'un générateur de vapeur, le milieu de refroidissement absorbant au moins une partie de la chaleur de réaction libérée dans la zone de réaction (b) et étant partiellement évaporé dans le procédé, et le mélange condensat-vapeur saturée résultant ou la vapeur saturée étant au moins partiellement ramené(e) au générateur de vapeur et/ou amené(e) comme caloporteur à la zone de préchauffage (a) de la même cellule de réaction.

9. Réacteur selon l'une des revendications précédentes, **caractérisé en ce qu'**il comporte des moyens permettant d'amener le mélange condensat-vapeur saturée évacué d'une ou plusieurs cellules de réaction ou seulement la partie vapeur au moins partiellement au générateur de vapeur et la vapeur saturée extraite du générateur de vapeur est au moins partiellement libérée vers des consommateurs externes en tant que vapeur d'exportation.

10. Réacteur selon l'une des revendications précédentes, **caractérisé en ce qu'**il comprend des moyens permettant, dans la zone de préchauffage (a), de chauffer le mélange d'alimentation ou le flux de produit gazeux provenant de la cellule de réaction amont par échange thermique indirect vers le condensat chaud provenant d'un générateur de vapeur, un flux de condensat chaud refroidi étant obtenu.

11. Réacteur selon l'une des revendications précédentes, **caractérisé en ce qu'**il comprend des moyens permettant d'amener le flux de condensat chaud, délivré par la zone de préchauffage (a) et refroidi, comme fluide de refroidissement à la première zone de refroidissement (c1) dans une cellule de réaction amont disposée en amont puis de le ramener au générateur de vapeur.

12. Réacteur selon l'une des revendications précédentes, **caractérisé en ce qu'**au moins une partie des zones de refroidissement et/ou des zones de préchauffage est réalisée sous forme d'échangeurs à plaques comportant des tôles thermiques.

13. Réacteur selon l'une des revendications précédentes, **caractérisé en ce qu'**il comprend des moyens permettant d'amener un mélange d'alimentation frais non encore partiellement converti ou des réactifs individuels à une ou plusieurs des cellules de réaction aval disposées en aval de la première cellule réactionnelle.

14. Réacteur selon l'une des revendications précédentes, **caractérisé en ce que** la zone de réaction (b) est équipée dans au moins une cellule réactionnelle d'au moins deux catalyseurs qui ont une activité différente quant à la réaction d'équilibre exothermique.

15. Utilisation d'un réacteur selon la revendication 1 à 14 pour produire du méthanol par réaction d'une charge de gaz de synthèse contenant du dioxyde de carbone.
